# EUROPEAN PATENT SPECIFICATION

(11) **EP 2 812 424 B1**
(45) Date of publication and mention of the grant of the patent: **28.08.2019**
(21) Application number: 13705237.9
(22) Date of filing: 07.02.2013
(51) Int. Cl.: C12M 1/32, B01L 3/00, C12M 3/00, C12M 1/12

(54) **MULTI-WELL PLATE**
MULTIWELL-PLATTE
PLAQUE MULTI-PUITS

(30) Priority: 07.02.2012 GB 201202138
(43) Date of publication of application: 17.12.2014
(73) Proprietor: The Electrospinning Company Ltd, Didcot OX11 0RL (GB)
(72) Inventor: MCKEAN, Robert James, Didcot OX11 0RL (GB)
(74) Representative: Icely, Dominic Michael
(86) International application number: PCT/GB2013/050280
(87) International publication number: WO 2013/117926

(56) References cited:
- WO-A1-2008/089708
- US-A1- 2002 155 617
- US-A1- 2005 047 971
- US-A1- 2005 095 695
- US-A1- 2008 206 343
- US-A1- 2009 246 864
- US-A1- 2010 112 690
- US-A1- 2010 233 115
- US-A1- 2011 244 572

## Description

### FIELD OF THE INVENTION

The present invention relates to a multi-well assay plate which is suitable for use in cell-based assays, and to a process for producing a multi-well assay plate.

### BACKGROUND TO THE INVENTION

Multi-well assay plates (also known as multi-well plates, microplates or microtiter plates) are known in the art and have a broad application in the life sciences, including in cell biology, microbiology, biophysics, pharmacology, toxicology and immunology, as well as other scientific fields. Typically, samples and reagents are stored, processed and/or analysed in multi-well plates.

Multi-well plates can take a variety of forms, shapes and sizes, and are particularly well suited for screening a number of samples at the same time. Standard sizes have appeared with the development of laboratory equipment that has been specifically designed for the multi-well plate format, including plate-readers, plate shakers, automated high throughput screening equipment, and the like. Thus, multi-well assay plates are made in standard sizes and shapes, having standard arrangements of wells and standard outer dimensions. The Society of Biomolecular Screening (SBS) has published recommended microplate specifications for a variety of plate formats. For example, SBS-standardised plates have the standardized outer dimensions of 14.25 mm in height, 85.48 mm in width and 127.76 mm in length. Some well-established arrangements of wells include those found on 96-well plates (12x8 array of wells), 384-well plates (24x16 array of wells) and 1536-well plates (48x32 array of wells).

In the drug discovery process, mammalian cells are routinely cultured in industry-standard 96-well, 384-well and 1536-well plates. Plates with higher numbers of wells are also used. The outer dimensions of these plates are typically identical so that they fit a range of measuring instruments and automated / robotic processing equipment, for example equipment for rapidly dispensing liquid in and out of the plates, equipment for rapidly measuring a signal (such as fluorescence, luminescence or optical absorbance), and equipment for storing and moving the plates. Cellular responses to drug stimulation are determined using colorimetric, fluorescence and bioluminescence based assays and commonly readouts are obtained using microplate readers and automated confocal microscopy.

Such cell-based assays are routinely used in high throughput screening (HTS), which is commonly used by pharmaceutical companies to assess the efficacy and toxicity of drug candidates. Simple assay readouts are used to determine the level of drug required to induce apoptosis, inhibit cell proliferation or decrease cell viability. Drug candidates are currently screened in two-dimensional cultures of cells (2D cell cultures) in 384 or 1536 well plates and assays are designed to be highly efficient and low cost due to the high number of potentially active compounds to be screened. High content screening (HCS) is also commonly used by pharmaceutical companies, to explore the action of a candidate drug on a cell in more detail, by carrying out multiple assays in a single well and using more complex analytical instruments such as confocal microscopy. Targets are commonly screened in 2D on 96 well plates.

A method for manufacturing such kind of multi-well assay plates which applies the infrared welding technique to attach an optically transparent base plate to a molded upper plate comprising an array of open ended wells is disclosed in US 2005/0047971.

In both HTS and HCS candidate drugs are typically screened in two-dimensional cultures of cells on the flat base of each well. Cells cultured in 2D on tissue culture plastic are flat, have 50% of their surface area exposed to tissue culture plastic, and 50% of their cell surface area exposed directly to cell culture media. This inhibits the production of extracellular matrix (ECM) which is responsible for signalling between cells over long distances and results in tissue specific gene expression. As a result, cells cultured in 2D are not genetically or phenotypically similar to their *in vivo* counterparts found in tissues, which comprise both cells and matrix molecules. Screening in 2D cell cultures can therefore lead to a high number of false positive results, which increases the number of drugs that fail only after expensive animal trials. Additionally, pre-clinical testing of novel compounds in model organisms such as mice and rats does not accurately represent how a drug may interact with cells in the more complex human physiological system. There is therefore an ongoing need to develop improved assay methods and equipment which facilitate more accurate *in vitro* drug discovery, and in particular which can reduce the number of false positives at an early stage.

### SUMMARY OF THE INVENTION

The present invention provides a multi-well plate which facilitates the growth of three-dimensional (3D) cell cultures with a high degree of consistency and reproducibility from well to well and from plate to plate. Moreover, the multi-well plate is capable of supporting 3D culture growth for a wide variety of cell types, including cell lines, stem cells and primary cells. Cells in 3D cultures have 100% of their cell surface area exposed to other cells and matrix. This stimulates specific signaling pathways to initiate tissue-specific gene expression and stimulate the production of extracellular matrix (ECM). As a result, the cultures grown in the multi-well plate of the invention are more similar to cells found within tissues in the body and provide a more accurate *in vitro* model for drug discovery than cells grown in 2D. Moreover such 3D cell cultures are more resistant to drug treatment than cells grown in 2D, meaning that the multi-well plate of the invention can advantageously reduce the number of false positives at the high throughput screening (HTS) and high content screening (HCS) stages and thereby reduce the number of drugs that fail only after undergoing animal trials. The invention therefore has applications in the 3Rs to reduce, refine and replace animal models with more accurate *in vitro* human tissue models. Furthermore, animal studies on model systems such as mice and rats do not accurately represent the more complex human physiological environment for which the drug is ultimately intended. At the same time, the multi-well plate of the invention is compatible with current automation equipment, with standard imaging equipment and with assays of the kind that are normally performed on 2D cell cultures. Standard fluorimetric assays can therefore be performed on the 3D cultures and the results can be measured using a standard plate reader or, for instance, by confocal microscopy. The assay plate of the invention therefore has the advantages of 3D cell culture growth but can otherwise be treated as if it were a standard 2D substrate, providing ease of use.

Accordingly, the invention provides a multi-well assay plate, which comprises:
a transparent plate base, which defines the bottom of a plurality of sample wells;
a scaffold layer disposed on the plate base, which scaffold layer provides a porous three-dimensional network of electrospun polymer nanofibres in each of said sample wells, wherein the thickness of the scaffold layer in each of said sample wells is from 10 µm to 150 µm and the mean diameter of the polymer nanofibres is from 500 nm to 10 µm; and
a plate frame, which defines the side walls of said sample wells;
wherein the plate frame is opaque and is welded to the plate base through the scaffold layer.

The invention also provides a process for producing a multi-well assay plate of the invention as defined above, which process comprises:
(a) disposing a scaffold layer between an opaque plate frame and a transparent plate base, wherein the plate frame defines side walls for a plurality of sample wells, the plate base defines a bottom for said plurality of sample wells, and the scaffold layer comprises a porous three-dimensional network of electrospun polymer nanofibres, wherein the thickness of the scaffold layer is from 10 µm to 150 µm and the mean diameter of the polymer nanofibres is from 500 nm to 10 µm; and
(b) welding the plate frame to the plate base, through the scaffold layer, by laser welding or by ultrasonic welding, to form a watertight or hermetic seal between the plate frame and the plate base.

The scaffold layer provides a porous three-dimensional network of polymer nanofibres in each well which supports 3D culture growth for a variety of cell types. The bond formed between the plate frame and the plate base seals the individual wells from one another, preventing cross-contamination between wells when this is not desired. At the same time it advantageously fixes the scaffold layer between the plate frame and the plate base at the same position at the base of each well. This ensures that each of the individual wells comprises a portion of the same porous network of polymer nanofibres, fixed at the same position in each well, providing for remarkable well-to-well consistency and reproducibility. In particular, use of the same scaffold layer in each well allows for consistent and reproducible 3D culture growth from well to well, and fixing that scaffold layer between the plate frame and the plate base ensures that the 3D cell cultures grown in the scaffold are fixed at the same distance from the top of the well. This ensures consistency of measurement from well to well when automated measuring equipment is used (such as a plate reader which measures fluorescence, or an automated confocal microscope). Fixing the scaffold in this way also ensures that the scaffold material does not become dislodged in a given well, which can lead to loss or dislodgement of the 3D cell culture, and/or the growth of 2D layers of cells on surfaces of the well which have become available or exposed by the dislodgement of the scaffold. Devices in which individual disks or pieces of scaffold material are placed separately in each well are not only more difficult and impractical to manufacture, but also do not provide for the same degree of consistency and reproducibility in cell growth and assay measurement from well to well, and the scaffolds are more susceptible to becoming dislodged. The use of a 3D network of nanofibres as opposed to alternative scaffold technology provides for greater consistency and reproducibility from batch to batch. For instance, it avoids the problems which are commonly associated with hydrogel scaffold materials, including low batch to batch reproducibility and incompatibility with current automation equipment.

The scaffold comprises a porous three dimensional network of polymer nanofibres which is produced by electrospinning, and in which the mean diameter of the polymer nanofibres is from 500 nm to 10 µm. The relative standard deviation from said mean is typically less than or equal to 15%, preferably less than 10%. Such scaffolds can be produced with consistent fibre diameters with a low relative standard deviation from the mean, and are reproducible from batch to batch. They therefore have a consistent porosity and pore size and have been found to be particularly useful for facilitating the growth of three-dimensional cell cultures for a variety of different cell types, consistently and reproducibly. Such scaffolds are particularly useful as the scaffold layer in the multi-well plates of the invention and, more generally, in tissue engineering applications.

The multi-well assay plate of the invention as defined above can be used in drug screening, regenerative medicine, and tissue engineering.

### BRIEF DESCRIPTION OF THE FIGURES

Fig. 1 is a schematic representation of an embodiment of the process of the invention for producing a multi-well assay plate of the invention, in which the plate frame is bonded to the plate base, through the scaffold layer, using laser welding.
Fig. 2 is a scanning electron microscope (SEM) image, at 500x magnification, of the poly(L-lactide) fibres of a scaffold produced by the electrospinning method described in Example 1 herein. The image was taken by a Phenom G2 pro scanning electron microscope.
Fig. 3 is an SEM image, at 1000x magnification, of the poly(L-lactide) fibres of a scaffold produced by the electrospinning method described in Example 1.
Fig. 4 is an SEM image, at 2000x magnification, of the poly(L-lactide) fibres of a scaffold produced by the electrospinning method described in Example 1.
Fig. 5 is a histogram of the fibre diameter distribution in a scaffold produced by electrospinning as described in Example 1 herein. The fibre diameter distribution was measured automatically, as described in Example 2, by the Fibremetric Software Application from PhenomWorld. The histogram was generated by the PhenomWorld software.
Fig. 6 is a chart showing the fibre diameter distribution in a scaffold produced by electrospinning as described in Example 1 herein. The column chart was generated in Excel, using data from the PhenomWorld software, as described in Example 2.

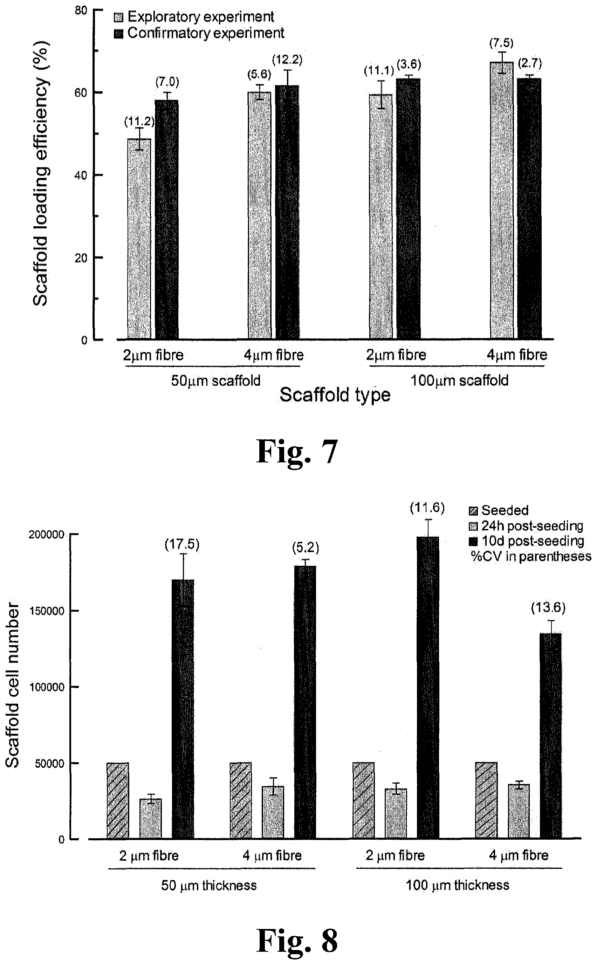

Fig. 7 is a bar chart showing the % cell loading efficiency (y axis) for (i) exploratory experiments, and (ii) confirmatory experiments 24 hours after cell seeding, on each of the following scaffold types: (a) 50 µm scaffold layer thickness, average fibre diameter of 2 µm; (b) 50 µm layer scaffold thickness, average fibre diameter of 4 µm; (c) 100 µm scaffold layer thickness, average fibre diameter of 2 µm; and (d) 100 µm scaffold layer thickness, average fibre diameter of 4 µm. The small coefficients of variance (% CV values), which are shown in parentheses adjacent the error bars, indicate that cell culture growth and cell loading efficiency are remarkably consistent and reproducible in the scaffolds of the invention.
Fig. 8 is a bar chart showing the scaffold cell number (y axis) for scaffolds of the invention (i) as seeded, (ii) 24 hours after seeding, and (iii) 10 days after seeding, for each of the following scaffold types: (a) 50 µm scaffold thickness, average fibre diameter of 2 µm; (b) 50 µm scaffold thickness, average fibre diameter of 4 µm; (c) 100 µm scaffold thickness, average fibre diameter of 2 µm; and (d) 100 µm scaffold thickness, average fibre diameter of 4 µm. Again, the low %CV values (shown in parentheses adjacent the error bars) are indicative of consistent and reproducible cell culture growth in scaffolds of the invention.

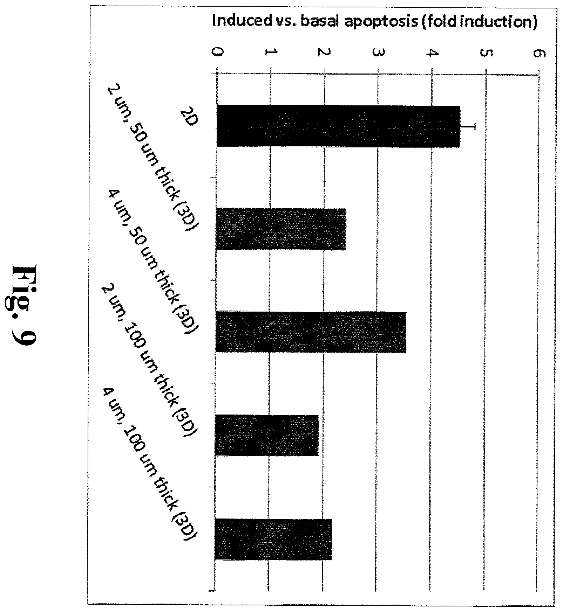

Fig. 9 is a bar chart showing induced versus basal apoptosis (fold induction) on the y axis, for (i) a conventional 2D cell culture (positive control), and (ii) 3D cell cultures in the following formats of scaffolds of the invention: (a) 50 µm scaffold thickness, average fibre diameter of 2 µm; (b) 50 µm scaffold thickness, average fibre diameter of 4 µm; (c) 100 µm scaffold thickness, average fibre diameter of 2 µm; and (d) 100 µm scaffold thickness, average fibre diameter of 4 µm. Growing in a 3D environment consistently protected the cells from apoptosis induction by staurosporine, a key determinant of the value of the scaffold culture environment.

### DETAILED DESCRIPTION OF THE INVENTION

The multi-well assay plate of the invention comprises: a transparent plate base, which defines the bottom of a plurality of sample wells; a scaffold layer disposed on the plate base, which scaffold layer provides a porous three-dimensional network of electrospun polymer nanofibres in each of said sample wells, wherein the thickness of the scaffold layer in each of said sample wells is from 10 µm to 150 µm and the mean diameter of the polymer nanofibres is from 500 nm to 10 µm; and a plate frame, which defines the side walls of said sample wells. The plate frame is opaque and is welded to the plate base through the scaffold layer.

The plate frame is typically a molded plastic frame, which defines the side walls of said plurality of sample wells. The plate frame also typically serves to provide the outer casing of the multi-well plate itself, meaning that its outer dimensions, particularly its length and width are usually the outer dimensions of the multi-well plate itself. Thus the outer dimensions of the plate frame may be standardized outer dimensions which allow the multi well plate of the invention to be used with automated / robotic processing equipment of the kind which is used in the pharmaceutical industry for drug screening. Such equipment includes apparatus for rapidly dispensing liquid in and out of the plate wells, for rapidly measuring a signal (such as fluorescence, luminescence or optical absorbance) and for storing and moving the plates. In one embodiment, the outer length and width of the plate frame are about 128 mm and about 85 mm respectively. The plate frame may for instance be 127.76 mm long and 85.48 mm wide; these are the SBS standardized length and width dimensions for multi-well plates. The height of the plate frame may for instance be about 14 mm, for instance 14.25 mm which is the SBS standardized height for a multi-well plate.

The length and width of the multi-well plate of the invention may be about 128 mm and about 85 mm respectively, for instance 127.76 mm long and 85.48 mm wide. The height of the multi-well plate of the invention may in some embodiments be about 14 mm, for instance 14.25 mm.

The plate frame defines the side walls of said plurality of sample wells. The number of said sample wells can be equal to or greater than two. More typically, however the number of said sample wells is equal to or greater than 6, or for instance equal to or greater than 24. Well plates having a larger number of wells, for instance 96-, 384- and 1536- well plates are commonly used in the pharmaceutical industry for drug screening. Thus, in preferred embodiments of the invention the plate frame defines the side walls of at least 96 wells. Or, for instance, the plate frame may define the side walls of at least 384 wells, or at least 1536 wells. In one embodiment, the number of said sample wells is selected from 96, 384, 1536 and 3456.

Thus, in one embodiment, the multi-well plate of the invention is a 96-well plate.

In another embodiment, the multi-well plate of the invention is a 384-well plate.

In another embodiment, the multi-well plate of the invention is a 1536-well plate.

In another embodiment, the multi-well plate of the invention is a 3456-well plate.

The sample wells are typically arranged in the plate frame in an array. Thus, in a 96-well plate, the sample wells are usually arranged in an array of 12 x 8 wells. In a 384-well plate, the sample wells are typically arranged in an array of 24 x 16 wells. In a 1536-well plate, the sample wells are usually arranged in an array of 48 x 32 wells. In a 3456-well plate, the sample wells are usually arranged in an array of 72 x 48 wells.

Accordingly, in one embodiment the multi-well plate is a 96-well plate and the sample wells are arranged in an array of 12 x 8 wells.

In another embodiment, the multi-well plate is a 384-well plate and the sample wells are arranged in an array of 24 x 16 wells.

In another embodiment, the multi-well plate is a 1536-well plate and the sample wells are arranged in an array of 48 x 32 wells.

In another embodiment, the multi-well plate is a 3456-well plate and the sample wells are arranged in an array of 72 x 48 wells.

The length and width of the array of sample wells are typically smaller than the values specified hereinbefore for the outer length and width of the plate frame and for the length and width of the multi-well plate itself. This is because the plate frame usually also comprises a "border" area around the edge of the array of sample wells, where no wells are present.

Thus, in a preferred embodiment the length of the array of sample wells is from 105 to 113 mm and the width of the array of sample wells is from 69 to 77 mm. More typically, in this embodiment, the length of the array of sample wells is about 109 mm and the breadth of the array of sample wells is about 73 mm. These dimensions are compatible with standard plate readers.

The plate frame is opaque, so that fluorescent assays may be performed on the multi-well plate without fluorescence from one sample well contaminating fluorescence measurements performed on another, adjacent sample well. Thus, typically the plate frame absorbs light at the wavelengths typically used for fluorescence-based assays. Thus, the plate frame is typically opaque to visible light. It is typically opaque to wavelengths in the range of from 390 to 710 nm. Accordingly, in preferred embodiments the plate frame is capable of absorbing visible light. In preferred embodiments the plate frame is capable of absorbing light having a wavelength of from 390 to 710 nm.

Usually, the plate frame is black in colour.

Another advantage of using an opaque plate frame is that it facilitates manufacture of the multi-well plate of the invention by a laser welding process, in order to bond the plate frame to the plate base through the scaffold layer. Lasers having a wavelength in the near-infrared region of the electromagnetic spectrum may be used in such laser welding processes, to heat and melt regions of the plate frame and thereby weld the plate frame to the plate base. Thus, in some embodiments the plate frame is capable of absorbing light in the near-infrared region of the electromagnetic spectrum. More typically, the plate frame is capable of absorbing light in both the visible and near-infrared regions of the electromagnetic spectrum. For instance, the plate frame may be capable of absorbing wavelengths in the range of from 390 nm to 1100 nm.

The plate frame is usually made of a polymer. Accordingly, in some embodiments, the plate frame comprises a polymer. Any suitable polymer may be used. Polystyrene is often employed.

If the plate frame is opaque, as described above, then typically the plate frame further comprises a pigment which renders the plate frame opaque. In one embodiment the pigment renders the plate frame opaque to light in the visible region of the electromagnetic spectrum. Thus, typically the pigment renders the plate frame opaque to wavelengths in the range of from 390 nm to 710 nm. More typically, the pigment renders the plate frame opaque to light in both the visible and near-infrared regions of the electromagnetic spectrum. Thus, for instance, the pigment may render the plate frame opaque to wavelengths in the range of from 390 nm to 1100 nm.

In some embodiments, therefore, the plate frame further comprises a pigment. The pigment may be one which is capable of absorbing light in the visible region of the electromagnetic spectrum. Thus, the pigment may absorb light having a wavelength in the range of from 390 nm to 710 nm. Alternatively, the pigment may be one which absorbs light in the both visible and the near infrared regions of the electromagnetic spectrum. For instance, the pigment may be one which absorbs light in the wavelength range of from 390 nm to to 1100 nm.

The pigment is usually a black pigment.

Thus, typically, the plate frame comprises a polymer and a pigment which renders the polymer opaque. Preferably, the plate frame comprises a polymer and a black pigment. Typically, the polymer is polystyrene.

The plate base defines the bottoms of the plurality of sample wells. It is typically designed for bottom-reading microplate readers for use in fluorescent assays, and is usually therefore formed by an unpigmented, flat sheet, which is typically transparent or translucent. The plate base is usually rectangular, to match the typical shape of the array of sample wells in the plate frame.

The plate base typically comprises a polymer. Typically, the plate frame and the plate base comprise the same polymer, although usually the plate base polymer does not comprise a pigment. Any suitable polymer may be used. Polystyrene is often employed. Thus, in one embodiment the plate base comprises polystyrene. The polystyrene is typically unpigmented polystyrene. Use of the same polymer in the plate base and the plate frame can aid bonding between the plate frame and the plate base, particularly if the plate frame is welded to the plate base.

In contrast to the plate frame, the plate base is transparent. Usually, the plate base is transparent to light at the wavelengths typically used for confocal microscopy and fluorescence-based assays. Thus, the plate base is usually transparent to light in the visible region of the electromagnetic spectrum. Thus, the plate base may be transparent to wavelengths of from 390 nm to 710 nm. More typically, the plate base is transparent to light in both the visible and near-infrared regions of the electromagnetic spectrum. Thus, for instance, the plate base may be transparent to wavelengths in the range of from 390 nm to 1100 nm. This not only facilitates use in confocal microscopy and fluorescence-based assays but also facilitates manufacture of the device by laser welding, as described further herein; in particular it can ensure that the laser does not heat and melt the plate base in the laser welding process.

The plate base defines the bottoms of the plurality of sample wells and therefore spans the entire area formed by the sample wells defined in the plate frame. For instance, when the plate frame comprises an array of sample wells of about 109 mm in length and about 73 mm in width, then the length of the plate base is usually at least about 109 mm and the width of the plate base is typically at least about 73 mm.

In one embodiment, the length of the plate base is at least about 105 mm, and the width of the plate base is at least about 69 mm. In another embodiment, the length of the plate base is at least about 109 mm and the width of the plate base is at least about 73 mm.

Typically, the lengths and widths of the plate base and the array of sample wells are substantially the same. Thus, in one embodiment, the length of the plate base is from 105 to 113 mm and the width of the plate base is from 69 to 77 mm. For instance, the length of the plate base may be about 109 mm and the width of the plate base may be about 73 mm.

The thickness of the plate base is typically from about 100 µm to about 700 µm. In some embodiments, for instance, the thickness of the plate base is about 170 µm. Advantageously, plate bases in this thickness range that are also transparent can be readily used in standard fluorescent assays of the kind commonly used in high throughput screening. For compatibility with confocal microscopy used in high content screening, the plate base must be 170 µm thick. The preferred material can be either polystyrene, polycarbonate, cyclic olefin copolymers or quartz glass.

In the multi-well plate of the invention, a scaffold layer is disposed on the plate base. The scaffold layer provides a porous three-dimensional network of electrospun polymer nanofibres in each of the sample wells.

Also, the plate frame is bonded to the plate base through the scaffold layer. In particular, the undersides of the side walls defined by the plate frame are bonded to the plate base, through the scaffold layer.

The plate frame is welded to the plate base. Depending on the nature of the bond between plate frame and plate base, and on the process used to form the bond and the nature of the polymer nanofibres in the scaffold layer, the nanofibres in the scaffold layer may or may not remain intact within said bond. For instance, if the bond between the plate frame and the plate base is formed by laser welding or ultrasonic welding (in which the undersides of the plate frame side walls are heated and melted at the point of contact between the plate frame and the scaffold layer, and then allowed to cool to form a weld between the plate frame and plate base) the nanofibres may or may not remain intact within the weld thus formed. In particular, depending on the melting point of the scaffold polymer, the nanofibres may or may not melt during the welding process at the point of contact between the plate frame and the scaffold layer. If the nanofibres melt, the nanofibres do not remain intact within the weld at the side walls of the wells, but rather the nanofibre polymer becomes part of the weld. In other cases, for instance if the nanofibres have a particularly high melting point, the nanofibres can remain intact within the weld.

Whichever kind of bond is formed between the plate frame and plate base, the scaffold layer remains intact within the sample wells themselves (as opposed to within the side walls of the sample wells) and thereby provides the same porous three-dimensional network of polymer nanofibres in each of the sample wells. This provides for consistent and reproducible 3D cell growth from well to well. At the same time, the bond formed between the plate frame and the plate base advantageously fixes the scaffold layer at the same position at the base of each well, providing further well to well consistency as explained further hereinbefore. The bond also ensures that the individual wells are sealed from one another, preventing cross-contamination between wells.

The plate frame is welded to the plate base.

Typically, the bond between the plate frame and the plate base forms a watertight seal between the plate frame and the plate base. In some embodiments the bond between the plate frame and the plate base provides a hermetic seal between the plate frame and the plate base. Thus, in some embodiments the plate frame is hermetically sealed to the plate base through the scaffold layer.

The scaffold layer in the multi-well plate of the invention provides a porous three-dimensional network of polymer nanofibres in each of the sample wells.

As used herein, the term "nanofibre" means a microscopic fibre whose diameter is conveniently measured in nanometres (nm) or micrometres (µm). The mean diameter of the nanofibres used in the present invention is from 500 nm to 10 µm.

The use of a porous three-dimensional network of polymer nanofibres as the scaffold material provides certain advantages. Batch to batch reproducibility is one such advantage, and compatibility with current automation equipment is another. It is a finding of the invention that networks of polymer nanofibres with specific mean fibre diameters and low standard deviations from the mean can be produced very consistently, for instance by electrospinning or by other suitable methods for producing non-woven fibrous networks, such as melt spinning, dry spinning, wet spinning and extrusion. Electrospinning has been found particularly suitable for consistent reproduction of nanofibre networks with desired mean fibre diameters and low standard deviations from the mean.

The present invention therefore provides for a high degree of control over the mean scaffold fibre diameter, and over the standard deviation from the mean, and scaffolds in which fibres have specific mean diameters with low variance from the mean can therefore be produced. This in turn provides control over the scaffold porosity and pore size, which, together with fibre diameter, are important factors that affect whether or not 3D cell cultures can successfully grow in the scaffold. As the skilled person will appreciate, a scaffold should be sufficiently porous, with large enough pores, to allow cells to infiltrate the scaffold and grow in 3D culture. On the other hand, the pore size cannot be too large as cells will then effectively fall through the pores and not fill the entire volume of the material; the material will not then be an effective scaffold. The porosity, average pore diameter and the average fibre diameter of a non-woven network are interrelated as explained, for instance in Greiner and Wendorff, Angew. Chem. Int. Ed. 2007, 46, 5670-5703. Thus, the present invention provides for a particularly high level of control over scaffold fibre diameter, porosity and pore size in order to facilitate the growth of a variety of cell types in 3D culture. Alternative technologies for 3D cell growth, on the other hand, including for instance hydrogel scaffolds, do not allow for such a high degree of control over such factors. It is particularly difficult to manufacture suitable hydrogel materials with desired porosities and pore sizes consistently from batch to batch. Batch to batch reproducibility is therefore problematic for such materials. Hydrogel scaffold materials are also incompatibile with current automation equipment.

Usually, the polymer nanofibres in the porous three-dimensional network of the scaffold are randomly oriented. In another embodiment, however, the nanofibres in the scaffold are aligned. An electrospinning process for aligned fibre production is for instance described in WO2011/011575, and such processes are also described in Z.-M. Huang et al., Composites Science and Technology 63 (2003) 2223-2253 and in Greiner and Wendorff, Angew. Chem. Int. Ed. 2007, 46, 5670-5703.

As mentioned above, the porous network of polymer nanofibres in the scaffold can be produced by electrospinning, as detailed further below, or by other suitable methods which are known to the skilled person including, but not limited to, melt spinning, dry spinning, wet spinning and extrusion. Electrospinning is preferred. Thus the polymer nanofibres in the network may comprise electrospun, melt-spun, dry-spun, wet-spun or extruded nanofibres. Usually, however, the nanofibres comprise electrospun nanofibres.

The scaffold layer in the multi-well plate of the invention provides a porous three-dimensional network of polymer nanofibres in each of the sample wells, which is effectively the same in each well. The mean diameter of the polymer nanofibres is usually therefore substantially the same in each of said sample wells, with a low standard deviation from said mean. The thickness of the scaffold layer is also usually the same in each of said sample wells. This provides a consistent scaffold structure from well to well and therefore reproducible 3D culture growth from well to well.

The mean diameter of the polymer nanofibres in the scaffold is from 500 nm to 10 µm. More typically, the mean diameter of the polymer nanofibres is from 800 nm to 5 µm, or for instance from 1 µm to 5 µm.

In a preferred embodiment, the mean diameter of the polymer nanofibres is from 1 µm to 3 µm. For instance, the mean diameter of the polymer nanofibres may be from 1.5 µm to 2.5 µm. Usually, in this embodiment the mean diameter of the polymer nanofibres is from 1.8 µm to 2.2 µm, for instance about 2.0 µm.

In another preferred embodiment, the mean diameter of the polymer nanofibres is from 3 µm to 5 µm. For instance, the mean diameter of the polymer nanofibres may be from 3.5 µm to 4.5 µm. Usually, in this embodiment the mean diameter of the polymer nanofibres is from 3.8 µm to 4.2 µm, for instance about 4.0 µm.

Typically, the relative standard deviation from said mean is less than or equal to 20%. Preferably, the relative standard deviation from said mean is less than or equal to 15%. For instance, the relative standard deviation from said mean may be less than or equal to 10%.

In a preferred embodiment, the mean diameter of the polymer nanofibres is from 1 µm to 3 µm, and the relative standard deviation from said mean is less than or equal to 15%. For instance, the mean diameter of the polymer nanofibres may be from 1.5 µm to 2.5 µm, and the relative standard deviation from said mean may be less than or equal to 15%. Usually, in this embodiment the mean diameter of the polymer nanofibres is from 1.8 µm to 2.2 µm, for instance about 2.0 µm, and the relative standard deviation from said mean is less than or equal to 15%.

In another preferred embodiment, the mean diameter of the polymer nanofibres is from 1 µm to 3 µm, and the relative standard deviation from said mean is less than or equal to 10%. For instance, the mean diameter of the polymer nanofibres may be from 1.5 µm to 2.5 µm, and the relative standard deviation from said mean may be less than or equal to 10%. Usually, in this embodiment the mean diameter of the polymer nanofibres is from 1.8 µm to 2.2 µm, for instance about 2.0 µm, and the relative standard deviation from said mean is less than or equal to 10%.

In yet another preferred embodiment, the mean diameter of the polymer nanofibres is from 3 µm to 5 µm, and the relative standard deviation from said mean is less than or equal to 15%. For instance, the mean diameter of the polymer nanofibres may be from 3.5 µm to 4.5 µm, and the relative standard deviation from said mean may be less than or equal to 15%. Usually, in this embodiment the mean diameter of the polymer nanofibres is from 3.8 µm to 4.2 µm, for instance about 4.0 µm, and the relative standard deviation from said mean is less than or equal to 15%.

The mean diameter of the polymer nanofibres may for instance be from 3 µm to 5 µm, and the relative standard deviation from said mean may be less than or equal to 10%. For instance, the mean diameter of the polymer nanofibres may be from 3.5 µm to 4.5 µm, and the relative standard deviation from said mean may be less than or equal to 10%. Usually, in this embodiment the mean diameter of the polymer nanofibres is from 3.8 µm to 4.2 µm, for instance about 4.0 µm, and the relative standard deviation from said mean is less than or equal to 10%.

Typically, the mean diameter of the polymer nanofibres in the scaffold is measured by Scanning Electron Microscopy (SEM). Usually, the relative standard deviation from said mean is also measured by SEM.

Automated image characterisation is generally performed using a Phenom Fibremetric SEM system (Lambda Photometrics), which enables the automated analysis of multiple images in order to determine the mean fibre diameter and the relative standard deviation. The Fibremetric software automatically identifies the location of the fibres due to the contrast within the captured SEM image and measures the diameter of each fibre 20 times at a specific location. Typically around 100 of such measurements are performed per image.

Alternatively, the diameter of the fibres can be obtained via manual measurements / analysis of multiple SEM images.

It is a finding of the invention that nanofibres with mean diameters in these ranges and with these standard deviations from the mean provide porous networks which facilitate the growth of three-dimensional (3D) cell cultures with a high degree of consistency and reproducibility from well to well and from plate to plate, for a variety of cell types.

The mean pore size in the scaffold layer is typically from 10 µm to 20 µm. Pore size can be difficult to measure accurately, though, as pore size depends on how far through the scaffold you measure and no two pores are the same shape due to the random orientation of the nanofibres. The pore size is tuned roughly to match a typical cell diameter (approx. 20 microns) however the loose nature of the nanofibres allows for cells to migrate into the scaffold by pushing the nanofibres aside. With respect to total porosity, the scaffolds typically have a porosity of greater than 75%. In other words, the scaffolds are typically greater than 75% air, by volume. The scaffolds may for instance have a porosity of greater than 80%. Typically, the porosity is from about 75% to about 85%. In some embodiments, however, the scaffolds may have a porosity of greater than 90%, for instance from about 90% to about 95%.

The thickness of the scaffold layer is from 10 µm to 150 µm. The thickness of the scaffold layer may for instance be from 30 µm to 120 µm, or for instance from 40 µm to 110 µm. More typically, the thickness of the scaffold layer is from about 50 µm to about 100 µm. Advantageously, scaffolds within these thickness ranges are generally thick enough to grow cell cultures which extend in all three dimensions enough to provide the benefits of a 3D cell culture versus a 2D layer of cells. As discussed hereinbefore, such benefits include more accurately mimicking *in vivo* conditions and thereby reducing false positives early on in the drug screening process. At the same time, cell cultures generated within such scaffolds are thin enough to render the culture easy to image using high throughput screening methods. Thicker cell cultures, for instance, can be time consuming to analyse by microscopy due to the need to collect more images in the z direction (i.e. a higher degree of "z-stacking" is necessary to image the sample) and such methods may therefore be less suitable for high throughput screening. Also, scaffolds within these thickness ranges are generally thin enough to manufacture very quickly and inexpensively using techniques such as electrospinning, and thin enough to facilitate rapid growth of 3D cell cultures. It can be time consuming and expensive to grow and then maintain 3D cell cultures in thick scaffolds. Another advantage with using scaffold layers within these thickness ranges is that it will be particularly easy to bond the plate frame to the plate base through such thin scaffold layers, by laser welding or ultrasonic welding.

The nanofibres in the scaffold layer typically comprise a biocompatible polymer. Any suitable biocompatible polymer can be employed, and the biocompatible polymer may for instance be a natural polymer or a synthetic polymer. In some embodiments, the polymer is a bioerodable polymer.

Preferably the polymer is one which has little or no autofluorescence. In particular, it is preferred that the polymer is one which has little or no autofluorescence in the wavelengths typically used for fluorescent assays, for instance at wavelengths in the visible region of the electromagnetic spectrum. The nanofibres in the scaffold layer typically therefore comprise a polymer which has little or no autofluorescence at wavelengths of from 390 nm to 710 nm.

The nanofibres in the scaffold layer may for instance comprise any of the following polymers:
poly(L-lactide); poly(glycolic acid); polyhydroxybutyrate; polystyrene; polyethylene; polypropylene; poly(ethylene oxide); a poly(ester urethane); poly(vinyl alcohol); polyacrylonitrile; polylactide; polyglycolide; polyurethane; polycarbonate; polyimide; polyamide; aliphatic polyamide; aromatic polyamide; polybenzimidazole; poly(ethylene terephthalate); poly[ethylene-co-(vinyl acetate)]; poly(vinyl chloride); poly(methyl methacrylate); poly(vinyl butyral); poly(vinylidene fluoride); poly(vinylidene fluoride-co-hexafluoropropylene); cellulose acetate; poly(vinyl acetate); poly(acrylic acid); poly(methacrylic acid); polyacrylamide; polyvinylpyrrolidone; poly(phenylene sulfide); hydroxypropylcellulose; polyvinylidene chloride, polytetrafluoroethylene, a polyacrylate, a polymethacrylate, a polyester, a polysulfone, a polyolefin, polysilsesquioxane, silicone, epoxy, cyanate ester, a bis-maleimide polymer; polyketone, polyether, polyamine, polyphosphazene, polysulfide, an organic/inorganic hybrid polymer or a copolymer thereof, for instance, poly(lactide-co-glycolide); polylactide-co-poly(ε-caprolactone) or poly(L-lactide)-co-poly(ε-caprolactone); or a blend thereof, for instance a blend of poly(vinyl alcohol) and poly(acrylic acid).

The nanofibres in the scaffold layer may comprise a bioerodible polymer, for instance a bioerodible polymer selected from poly(L-lactide); poly(glycolic acid); polyhydroxybutyrate; and poly(ester urethanes).

The nanofibres in the scaffold layer may alternatively for instance comprise a biopolymer, or a blend of a biopolymer with a synthetic polymer. The following biopolymers and blends of biopolymers with synthetic polymers may for instance be used:
collagen; collagen/poly(ethylene oxide); collagen/poly(ε-caprolactone); collagen/polylactide-co-poly(ε-caprolactone); gelatin; gelatin/poly(ε-caprolactone); gelatin/ poly(ethylene oxide); casein/poly(vinyl alcohol); casein/poly(ethylene oxide); lipase; cellulase/poly(vinyl alcohol); bovine serum albumin/poly(vinyl alcohol); luciferase/poly(vinyl alcohol); α-chymotrypsin; fibrinogen; silk; regenerated silk; regenerated *Bombyx mori* silk; Bombyx mori silk/poly(ethylene oxide); silk fibroin; silk fibroin/chitosan; silk fibroin/chitin; silk/poly(ethylene oxide) (coaxial); artificial spider silk; chitin; chitosan; chitosan/poly(ethylene oxide); chitosan/poly(vinyl alcohol); quaternized chitosan/poly(vinyl alcohol); hexanoylchitosan/polylactide; cellulose; or cellulose acetate.

The nanofibres in the scaffold layer may alternatively for instance comprise a blend of two or more polymers, a copolymer (which may for instance be a block copolymer), or a blend of a polymer with an inorganic material.

Non-limiting examples of such blends blend of two or more polymers include a polyvinylpyrrolidone/polylactide blend; a polyaniline/polystyrene blend; a polyaniline/poly(ethylene oxide) blend; a poly(vinyl chloride)/polyurethane blend, a poly[(m-phenylene vinylene)-co-(2,5-dioctyloxy-p-phenylene vinylene)]/poly(ethylene oxide) blend; a poly[2-methoxy-5-(2'-ethylhexyloxy)-1,4-phenylene vinylene] (MEH-PPV)/polystyrene blend, a polyaniline/polystyrene blend; a polyaniline/polycarbonate blend, a poly(ethylene terephthalate)/ poly(ethylene terephthalate)-co-poly(ethylene isophthalate) blend, a polysulfone/polyurethane blend; a chitosan/polylactide blend, a polyglycolide/chitin blend, and a polylactide/poly(lactide-co-glycolide) blend.

Non-limiting examples of such block copolymers systems include polylactide-b-poly(ethylene oxide) block copolymers; poly(lactide-co-glycolide)-b-poly(ethylene oxide) block copolymers; poly[(trimethylene carbonate)-b-(ε-caprolactone)] block copolymers; polystyrene-b-polydimethylsiloxane and polystyrene-b-polypropylene block copolymers; polystyrene-b-polybutadiene-b-polystyrene block copolymers and polystyrene-b-polyisoprene block copolymers.

Non-limiting examples of blends of a polymer with an inorganic material from which fibres can be produced include: montmorillonite with polyamide 6, polyamide 6,6 and poly(vinyl alcohol), poly(methyl methacrylate), or polyurethane as the carrier material; a blend of a polymer carrier and noble metal nanoparticles, for instance poly(acrylonitrile)-co-poly(acrylic acid)/Pd; poly(ethylene oxide)/Au; polyvinylpyrrolidone/Ag; and poly(acrylonitrile)/Ag; a blend of a polymer carrier and magnetic nanoparticles, for instance poly(ethylene oxide) (or poly(vinyl alcohol))/Fe₃O₄, poly(ε-caprolactone)/FePt; polyurethane/MnZnNi; and poly(methyl methacrylate)/Co; a blend of a polymer and carbon nanotubes, for instance carbon nanotubes blended with poly(acrylonitrile), poly(ethylene oxide), poly(vinyl alcohol), polylactide, polycarbonate, polystyrene, polyurethane, or poly(methyl methacrylate); a blend of a polymer and a metal oxide or metal sulfide, for instance: polymer/TiO₂ wherein the polymer may for instance be with polyvinylpyrrolidone, poly(vinyl acetate), or poly(acrylonitrile); polymer/ZrO₂ wherein the polymer may for instance be polyvinylpyrrolidone, poly(vinyl acetate) and poly(vinyl alcohol); and blends of a polymer or polymers with: ZnO, CuO, NiO, CeO₂, Mn₃O₄, Mn₂O₃/Mn₃O₄, MoO₃, BaTiO₃, Y₂O₃, Gd₂O₃, Ta₂O₅, CO₃O₄, Ba_{0.6}Sr_{0.4}TiO₃, SiO₂, CdS, PbS, and Ag₂S.

Thus, the nanofibres in the scaffold layer may for instance comprise any of the materials listed in the preceding paragraphs. Scaffolds of nanofibers comprising the above polymers, copolymers, blends two or more polymers, and blends of a polymer with an inorganic material, can be produced by electrospinning, as detailed in in Greiner and Wendorff, Angew. Chem. Int. Ed. 2007, 46, 5670-5703.

In a preferred embodiment, the nanofibres in the scaffold layer comprise poly(L-lactide). It is a finding of the invention that scaffold layers comprising a porous, three-dimensional network of poly(L-lactide) nanofibres are particularly suitable for manufacturing the multi-well assay plate of the invention, owing to the advantageous handling properties of the scaffold material, the biocompatibility and biodegradability of the polymer, the low autofluorescence of the polymer, and the ability of such scaffolds to support 3D cell culture growth for a variety of cell types. Sheets of scaffold material comprising poly(L-lactide) nanofibres were found to handle particularly well, allowing for ease of manufacture of the multi-well plate of the invention. The material was found to handle like a sheet of paper, i.e. it is flexible but not brittle or weak, and also not stretchy or elastic. Poly(L-lactide) is also biocompatible and biodegradable, and is therefore a suitable polymer for use in scaffolds for growing three-dimensional cell cultures. A further advantage of poly(L-lactide) is that the scaffold layers which comprise poly(L-lactide) used in the present invention have little or no autofluorescence in the wavelengths typically used for fluorescent assays.

Usually, therefore, the nanofibres in the scaffold layer comprise poly(L-lactide). The nanofibres in the scaffold layer may comprise a further polymer, or an inorganic material, in addition to the poly(L-lactide) polymer. Such further polymers and inorganic materials can be selected from those listed hereinbefore. Typically, however, the nanofibres in the scaffold layer in the multi-well plate of the invention contain only poly(L-lactide), or at least substantially only poly(L-lactide). Thus, usually, the nanofibres in the scaffold material consist essentially of poly(L-lactide). For instance, the nanofibres may consist of poly(L-lactide).

Typically, the poly(L-lactide) is poly(L-lactide) which has an inherent viscosity midpoint of from 1.0 dl/g to 2.5 dl/g. The poly(L-lactide) may for instance have an inherent viscosity midpoint of from 1.5 dl/g to 2.1 dl/g, for instance an inherent viscosity midpoint of about 1.8 dl/g.

The poly(L-lactide) typically has a weight average molecular weight (M_{w}) of from 50,000 g/mol to 250,000 g/mol. Preferably, the poly(L-lactide) has a M_{w} of from 120,000 g/mol to 180,000 g/mol, for instance about 150,000 g/mol.

The scaffold layer provides a porous three-dimensional network of polymer nanofibres in each of the sample wells and the scaffold layer therefore needs to span the entire area formed by the sample wells defined in the plate frame. The scaffold layer is usually rectangular, to match the typical shape of the array of sample wells in the plate frame.

Thus, when the plate frame comprises an array of sample wells of about 109 mm in length and about 73 mm in width, then the length of the scaffold layer is usually at least about 109 mm and the width of the scaffold layer is typically at least about 73 mm.

In one embodiment, the length of the scaffold layer is at least about 105 mm, and the width of the scaffold layer is at least about 69 mm. In another embodiment, the length of the scaffold layer is at least about 109 mm and the width of the scaffold layer is at least about 73 mm.

In a preferred embodiment the length of the scaffold layer is from 105 to 113 mm and the width of the scaffold layer is from 69 to 77 mm. More typically, in this embodiment, the length of the scaffold layer is about 109 mm and the width of the scaffold layer is about 73 mm.

Typically, the lengths and widths of the scaffold layer and the array of sample wells are substantially the same. Thus, in one embodiment, the lengths of the scaffold layer and the array of sample wells are from 105 to 113 mm and the widths of the scaffold layer and the array of sample wells are from 69 to 77 mm. For instance, the lengths of the scaffold layer and the array of sample wells may be about 109 mm and the widths of the scaffold layer and the array of sample wells may be about 73 mm.

Typically, the lengths and widths of the scaffold layer and the plate base are substantially the same. Thus, in one embodiment, the lengths of the scaffold layer and the plate base are from 105 to 113 mm and the widths of the scaffold layer and the plate base are from 69 to 77 mm. For instance, the lengths of the scaffold layer and the plate base may be about 109 mm and the widths of the scaffold layer and the plate base may be about 73 mm.

Typically, the lengths and breadths of the plate base, the scaffold layer and the array of sample wells are substantially the same. Thus, in one embodiment, the lengths of the plate base, the scaffold layer and the array of sample wells are from 105 to 113 mm and the widths of the plate base, the scaffold layer and the array of sample wells are from 69 to 77 mm. For instance, the lengths of the plate base, the scaffold layer and the array of sample wells may be about 109 mm and the widths of the plate base, the scaffold layer and the array of sample wells may be about 73 mm.

Such dimensions are compatible with standard plate readers and the like.

The multi-well plate of the invention is capable of supporting 3D culture growth for a wide variety of cell types, including cell lines, stem cells and primary cells.

Accordingly, in some embodiments the multi-well plate further comprises cells attached to the scaffold layer. The cells may for instance be a cell line, stem cells or primary cells. The cells are typically mammalian cells. The mammalian cells may for instance be a cell line, stem cells or primary cells.

The multi-well plate of the invention may for instance further comprise a 3D cell culture within said scaffold layer. Typically the 3D cell culture comprises cells, for instance mammalian cells, and extracellular matrix. The cells may for instance be a cell line, stem cells or primary cells. Typically, the cells are mammalian cells selected from a mammalian cell line, mammalian stem cells or mammalian primary cells.

The multi-well assay plate of the invention has the advantages of 3D cell culture growth but can otherwise be treated as if it were a standard 2D substrate. It is therefore useful for drug screening. In particular it is useful in high throughput drug screening using 3D cell cultures, and in high content screening using 3D cell cultures.

Accordingly, the invention further provides the use of the multi-well assay plate of the invention as defined above in drug screening. Typically, said drug screening is high throughput screening. In another embodiment, the drug screening is high content screening.

The multi-well assay plate is also useful in research relating to regenerative medicine (i.e. the process of replacing or regenerating human cells, tissues or organs to restore or establish normal function) and tissue engineering research. Accordingly, the invention also provides the use of the multi-well assay plate of the invention as defined above in regenerative medicine. Further provided is the use of the multi-well assay plate of the invention as defined above in tissue engineering.

Multi-well assay plates of the invention as defined hereinbefore can be produced by the process of the invention for producing a multi-well assay plate, which process comprises:
(a) disposing a scaffold layer between an opaque plate frame and a transparent plate base, wherein the plate frame defines side walls for a plurality of sample wells, the plate base defines a bottom for said plurality of sample wells, and the scaffold layer comprises a porous three-dimensional network of electrospun polymer nanofibres, wherein the thickness of the scaffold layer is from 10 µm to 150 µm and the mean diameter of the polymer nanofibres is from 500 nm to 10 µm; and
(b) welding the plate frame to the plate base, through the scaffold layer, by laser welding or by ultrasonic welding, to form a watertight or hermetic seal between the plate frame and the plate base.

The process of the invention for producing a multi-well plate comprises disposing a scaffold layer between an opaque plate frame and a transparent plate base.

The plate frame is typically as defined hereinbefore for the multi-well assay plate of the invention, for instance it is usually a molded plastic frame which defines the side walls of said plurality of sample wells, and it typically has the dimensions and well array structures specified above for use on standard equipment. The plate base may therefore be as further defined hereinbefore for the multi-well assay plate of the invention.

Similarly, the plate base is typically as defined hereinbefore for the multi-well assay plate of the invention, for instance it is typically designed for bottom-reading microplate readers for use in fluorescent assays, and is usually therefore formed by an unpigmented, flat sheet, which is transparent. The plate base is usually a rectangular sheet, to match the typical shape of the array of sample wells in the plate frame. The plate base may therefore be as further defined hereinbefore for the multi-well assay plate of the invention.

The scaffold layer comprises a porous three-dimensional network of polymer nanofibres, and this is also typically as defined hereinbefore for the multi-well assay plate of the invention. Thus, the thickness of the scaffold layer is from 10 µm to 150 µm, or for instance from 30 µm to 120 µm. Usually it is from 40 µm to 110 µm, or more typically, for instance, from about 50 µm to about 100 µm. The nanofibres in the scaffold layer may comprise any of the polymers, copolymers and blends defined above for the multi-well assay plate of the invention. Moreover, the polymer is preferably one which displays little or no autofluorescence when employed in a scaffold layer. The polymer nanofibres usually have a mean diameter and a relative standard deviation from the mean as defined above. Thus, the mean diameter of the polymer nanofibres in the scaffold layer is from 500 nm to 10 µm. More typically, it is from 800 nm to 5 µm, or for instance from 1 µm to 3 µm. In some embodiments, the mean diameter of the polymer nanofibres is from 1.5 µm to 2.5 µm. For instance, the mean diameter of the polymer nanofibres may be from 1.8 µm to 2.2 µm, or, for example, about 2.0 µm. Typically, the relative standard deviation from said mean is less than or equal to 15%. More typically, the relative standard deviation is less than or equal to 10%. In some embodiments, the relative standard deviation from said mean is less than or equal to 8%, for instance less than or equal to 5%. Usually, the polymer nanofibres are randomly oriented in the scaffold, but they may be aligned. The scaffold layer is usually a rectangular sheet, to match the typical shape of the array of sample wells in the plate frame. The scaffold layer may therefore be as further defined hereinbefore for the multi-well assay plate of the invention.

Such porous networks of electrospun polymer nanofibres in the scaffold are produced by electrospinning. The production of the scaffold layer by electrospinning is described further below as an optional part of the process of the invention for producing a multi-well plate.

The step of disposing the scaffold layer between the plate frame and the plate base typically involves contacting the scaffold layer with the plate frame and the plate base, wherein the scaffold layer is disposed between the plate frame and plate base. It also usually involves applying a force to hold these components together while the step of bonding the plate frame to the plate base, through the scaffold layer, is performed. This may for instance involve clamping the scaffold layer between the plate frame and the plate base.

The step of bonding the plate frame to the plate base typically involves bonding the undersides of the side walls defined by the plate frame to the plate base, through the scaffold layer.

The step of bonding the plate frame to the plate base may comprise any suitable method of forming a bond or attachment between the plate frame and the plate base, through the scaffold layer. Usually, the bonding involves forming a watertight seal or a hermetic seal between the plate frame and the plate base, through the scaffold layer, so that adjacent wells are sealed from one another. This ensures that the contents of one well do not leak into another well when the multi-well plate is in use, and therefore prevents undesired cross-contamination of wells.

Typically, therefore, said bonding (b) comprises forming a water-tight seal between the plate frame and the plate base, through the scaffold layer. More typically, said bonding (b) comprises forming a hermetic seal between the plate frame and the plate base, through the scaffold layer.

The plate frame is welded to the plate base.

Thus, said bonding (b) comprises welding the plate frame to the plate base, through the scaffold layer. Typically, said welding comprises laser welding or ultrasonic welding.

Typically, said bonding (b) comprises:
heating the plate frame and thereby melting the plate frame at the point of contact between the plate frame and the scaffold layer; and
allowing the melt to cool and solidify, to form a weld between the plate frame and the plate base.

As the skilled person will understand, the step of heating the plate frame generally involves heating only the underside of the plate frame (specifically, the undersides of the side walls defined by the plate frame) so that the plate frame melts only at the point of contact between the plate frame and the scaffold layer. Thus, typically only the undersides of the side walls defined by the plate frame are melted.

When the plate frame melts, the liquid melt may penetrate through the porous scaffold layer to contact the plate base, and then, upon cooling, solidify to form a bond between the plate frame and plate base, through the scaffold layer. Alternatively, the step of heating the plate frame may also cause the scaffold layer to melt locally, at the point of contact with the plate frame. The melted plate frame and locally melted scaffold may then solidify to form said weld between the plate frame and plate base, through the scaffold layer.

Usually, said heating melts both the plate frame and the scaffold layer at the point of contact between the plate frame and the scaffold layer, and the step of allowing the melt to cool results in the formation of said weld. It is thought that the locally melted scaffold layer may also have adhesive qualities which may strengthen the bond between the plate frame and the plate base.

Typically, the plate frame is heated by exposing it to light. The light may for instance be near infrared light, for instance light having a wavelength of from 810 to 1064 nm. Light having a wavelength of about 940 nm may for instance be employed.

Typically, said light is laser light. Accordingly, the plate frame is usually heated by exposing it to a laser. The laser may for instance be an infrared laser. The wavelength of the laser may for instance be in the range 810 to 1064 nm, for example about 940 nm. Typically a diode laser is used with a wavelength of 940 nm.

Typically, when the plate frame is heated by exposing it to light, for instance to a laser, a photo mask is used to ensure that the plate frame is exposed to the light, and therefore heated and melted, only at the point of contact between the plate frame and the scaffold layer (i.e. only at the bases of the side walls defined by the plate frame, that are to be welded to the plate base). The photo mask prevents said light from reaching areas of the scaffold layer disposed between the side walls, within the wells; those parts of the scaffold layer cannot be allowed to melt as they need to remain intact in the final product, so that the scaffold layer provides the porous 3D network of polymer nanofibres in each of said wells. The individual wells in the final product will therefore contain unaltered porous 3D network of polymer nanofibres. The patterned photo mask may for instance be a copper/quartz mask or a chrome/borosilicate mask.

Alternatively, the plate frame may be heated by ultrasonic energy. Ultrasonic energy can be used to vibrate the plate frame relative to the scaffold layer and plate base, thereby causing the plate frame to be heated, and melted, locally at the point of contact between the plate frame and the scaffold layer. The melt is then allowed to cool to form a bond between the plate frame and plate base, through the scaffold layer.

Usually, however, the plate frame is heated by exposing it to light, typically by exposing it to a laser. Typically, therefore the process of the invention for producing a multi-well assay plate comprises the following steps:
(a1) contacting a scaffold layer with a plate frame and a plate base, wherein the scaffold layer is disposed between the plate frame and plate base, wherein the plate frame defines side walls for a plurality of sample wells, the plate base defines a bottom for said plurality of sample wells, and the scaffold layer comprises a porous three-dimensional network of polymer nanofibres;
(a2) applying a patterned photo mask to the outside of the plate base and applying a force to hold the scaffold layer and plate base between the plate frame and the patterned photo mask; and
(b1) exposing the patterned photo mask to light and thereby heating and melting said plate frame at the point of contact between the plate frame and the scaffold layer,
   wherein the patterned photo mask substantially prevents said light from reaching areas of the scaffold layer between the side walls, and wherein the plate base is transparent to said light; and
(b2) allowing the melt to cool to form a weld between the plate frame and the plate base through the scaffold layer.

Typically, the process further comprises: (b3) removing the patterned photo mask.

A further, protective layer can be applied to the outside of the plate base, before the patterned photo mask is applied; this ensure that the plate base is not damaged during the laser welding process and that the final multi-well plate product has a smoother, flatter base.

Thus, the process typically comprises the following steps:
(a1) contacting a scaffold layer with a plate frame and a plate base, wherein the scaffold layer is disposed between the plate frame and plate base, wherein the plate frame defines side walls for a plurality of sample wells, the plate base defines a bottom for said plurality of sample wells, and the scaffold layer comprises a porous three-dimensional network of polymer nanofibres;
(a2) applying a protective layer to the outside of the plate base;
(a3) applying a patterned photo mask to the outside of the protective layer and applying a force to hold the scaffold layer, plate base, and protective layer between the plate frame and the patterned photo mask; and
(b1) exposing the patterned photo mask to light, and thereby heating and melting said plate frame at the point of contact between the plate frame and the scaffold layer,
   wherein the patterned photo mask substantially prevents said light from reaching areas of the scaffold layer disposed between the side walls, and wherein the plate base and the protective layer are transparent to said light; and
(b2) allowing the melt to cool to form a weld between the plate frame and the plate base through the scaffold layer.

Typically, the process further comprises (b3) removing the patterned photo mask and the protective layer.

The protective layer is usually an unpigmented, transparent, plastic sheet, for instance a clear polystyrene sheet.

Typically, the protective layer covers at least the whole area of the plate base to which it is applied. The thickness of the protective layer is usually at least 50 µm. The thickness of the protective layer may for instance be from 50 µm to 300 µm, for instance about 150 µm. Typically the protective layer comprises a polymer. Any suitable polymer may be employed. Typically, however the protective layer is made of the same material as the plate base. Thus, usually, the protective layer comprises polystyrene.

The light to which the patterned photo mask is exposed in the embodiments of the process described above may for instance be near infrared light, for instance light having a wavelength of from 810 to 1064 nm. Light having a wavelength of about 940 nm may for instance be employed.

Any suitable patterned photo mask may be employed, but the mask is usually a copper/quartz mask or a chrome/borosilicate mask.

Typically, said light is laser light. Accordingly, the plate frame is usually heated by exposing it to a laser. Thus, step (b1) of the embodiments of the process described above typically comprises exposing the patterned mask to a laser and thereby heating and melting said plate frame at the point of contact between the plate frame and the scaffold layer. The laser may for instance be a near infrared laser. The wavelength of the laser may for instance be in the range 810 to 1064 nm, for example about 940 nm. Typically a diode laser is used with a wavelength of 940 nm.

The step of exposing the patterned photo mask to a laser typically comprises scanning a laser beam across the exposed surface of the photo mask. The scanning rate must not be too slow, as that increases the likelihood of excessive heating and melting of the plate frame. It must also not be too fast, as in that case the plate frame may not be heated enough to melt and form an adequate weld, and regions of the plate frame may remain unsealed to the plate base. Scanning rates of from about 150 mm/min to about 250 mm/min have been found to be useful depending on the materials and laser used. Thus, the step of exposing the patterned photo mask to a laser may comprise scanning a laser beam across the exposed surface of the photo mask at a rate of from 150 mm/min to 250 mm/min. The scanning rate may for instance be from 190 mm/min to 210 mm/minm, for instance about 200 mm/min. As the skilled person will appreciate, the step of scanning a laser beam across the exposed surface of the photo mask could involve either (i) moving the photo mask/plate base/scaffold layer/plate frame assembly past a stationary laser, or (ii) moving a laser across the stationary photo mask/plate base/scaffold layer/plate frame assembly, or (iii) moving both the laser and the assembly. In all cases, the laser is scanned across the exposed surface of the photo mask.

After the step of bonding the plate frame to the plate base, the process of the invention for producing a multi-well assay plate may optionally further comprise: (c) sterilising the multi-well plate. The step of sterilising the multi-well plate typically comprises exposing the multi-well plate to gamma radiation.

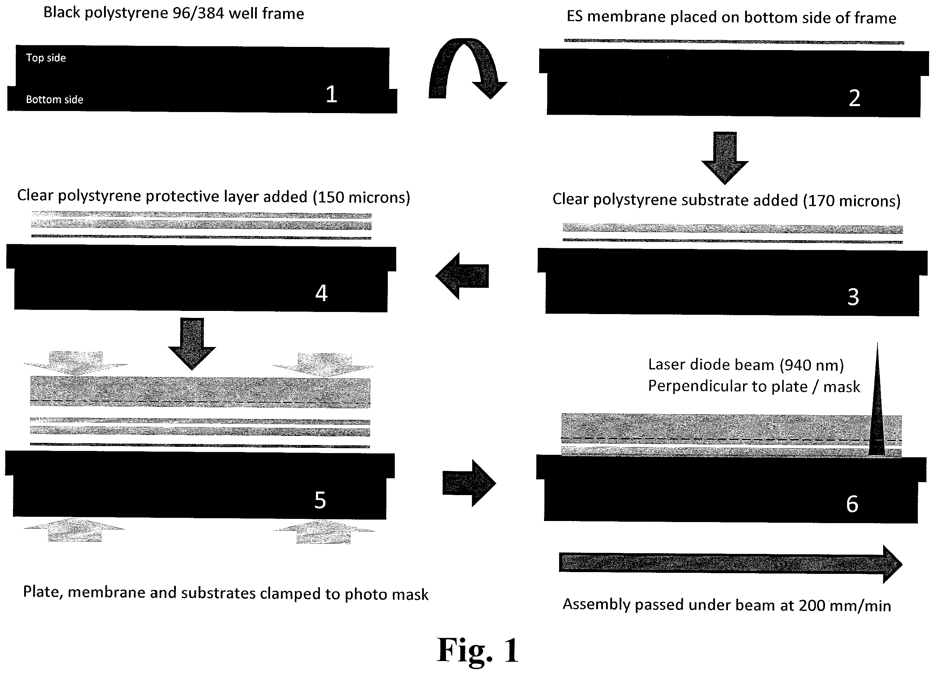

An exemplary laser welding process is shown schematically in Fig. 1, in which step 1 depicts a cross section of a black polystyrene 96-well or 384-well plate frame. In step 2, the scaffold layer, which is an electrospun membrane comprising a porous network of poly(L-lactide) nanofibres, is place on the underside of the plate frame, so that it contacts the undersides of the side walls of the wells. In step 3, a clear polystyrene plate base, having a thickness of about 170 µm, is disposed on the scaffold layer, so that the scaffold layer is sandwiched between the plate frame and plate base. A clear polystyrene protective layer, having a thickness of about 150 µm, is then disposed on the plate base in step 4. Then, in step 5, the plate frame, scaffold layer, plate base and protective layer are clamped to a photo mask, so that the photo mask is in contact with the protective layer. Step 6 shows the photo mask/protective layer/plate base/scaffold layer/plate frame assembly being passed under a laser diode beam at a rate of 200 mm/min, wherein the laser diode beam is disposed perpendicularly to the assembly and has a wavelength of 940 nm.

The scaffold layer, which comprises said porous three-dimensional network of polymer nanofibres, can be produced by electrospinning or by other suitable methods for producing non-woven fibrous networks, such as melt spinning, dry spinning, wet spinning and extrusion.

It is a finding of the invention however that electrospinning is particularly suitable for consistent reproduction of scaffold layers, for use in multi-well plates, which have specific desired mean fibre diameters and low standard deviations from the mean. Thus, electrospinning provide for high batch-to-batch reproducibility. It also allows the properties of the fibrous networks (in particular the fibre diameter, and therefore porosity and pore size) to be tuned to suit the growth of particular cell types, and for such tailored scaffolds to be produced consistently with high batch-to-batch reproducibility.

The process of electrospinning *per se* is well known, and is described for instance in the following review articles: Z.-M. Huang et al., Composites Science and Technology 63 (2003) 2223-2253 and in Greiner and Wendorff, Angew. Chem. Int. Ed. 2007, 46, 5670-5703.

Typically, a polymer or polymer blend from which a fibrous network is to be produced is dissolved in an appropriate solvent until a homogeneous solution of the required concentration is obtained. The concentration of the polymer solution must generally be high enough to achieve adequate chain entanglements in order for a continuous fibre to be formed. The polymer solution is typically then loaded into a vessel, usually a syringe, connected to a conductive (typically metal) capillary. The capillary is connected to a high voltage (usually to the positive terminal of a high voltage DC power supply), at a fixed distance from an earthed collection device. The collection device may be metallic, and is typically covered in a collection substrate onto which the fibres are deposited. The collection device is preferably rotatable, to ensure uniform deposition of the material. Fibres are typically produced by passing the polymer solution at a fixed flow rate through the metal capillary whilst applying a high voltage to the capillary in order to establish an electric field between the capillary and the collection device. The applied voltage should be high enough to overcome the surface tension of the polymer droplet at the tip of the capillary. As the charge builds at the surface of the droplet, the surface area has to increase to accommodate the additional charge, this occurs through the formation of a Taylor Cone from the droplet, from which a continuous fibre is extracted. As the fibre travels towards the grounded collector, the solvent rapidly evaporates and the fibre is further elongated due to instabilities arising from the columbic repulsions of the surface charges on the jet. The instabilities in the jet resulting from the high charge density cause the jet to whip about rapidly resulting in a nano/micro diameter solid (dry) filament. The collector is rotated slowly (at a rate of around 100 rpm) resulting in the deposition of a non-woven fibrous membrane on the substrate. After a fixed amount of material has been deposited to generate a layer or membrane of a particular desired thickness, the layer or membrane is dried in order to remove any residual solvent/moisture from the fibres. Typically, it is dried under vacuum, for instance for 24-48 hours at room temperature (approx. 25 °C).

Typically, therefore, the process of the invention for producing a multi-well plate further comprises: producing the scaffold layer by electrospinning a nanofibre precursor solution onto a collection substrate, wherein the nanofibre precursor solution comprises said polymer dissolved in a solvent.

Usually, said electrospinning comprises:
providing a fibre forming module adjacent a fibre collection device and spaced therefrom, wherein the fibre forming module comprises a dispensing capillary and wherein the fibre collection device is earthed and comprises a collection substrate;
applying a voltage across the dispensing capillary and the fibre collection device;
and, whilst applying said voltage,
feeding (preferably pumping) said nanofibre precursor solution through the dispensing capillary, thereby causing deposition of said scaffold layer on the collection substrate.

Any suitable polymer may be employed in the nanofibre precursor solution used in the electrospinning process. The polymer employed may be any of the polymers listed above in relation to the scaffold layer in the multi-well plate of the invention, or any of the copolymers, blends of two or more polymers, and blends of a polymer with an inorganic material listed hereinbefore. All of those polymers, copolymers and blends can be used in an electrospinning process to produce a porous three dimensional network of nanofibres, as detailed in Greiner and Wendorff, Angew. Chem. Int. Ed. 2007, 46, 5670-5703. The polymer used is generally of course a biocompatible polymer, and may for instance be a natural polymer or a synthetic polymer. In some embodiments, the polymer is a bioerodable polymer. Also, the polymer is preferably one which has little or no autofluorescence.

Preferably, however, the polymer comprises poly(L-lactide).

Usually, the polymer is poly(L-lactide). Thus, the polymer may consist essentially of poly(L-lactide), or, for instance, the polymer may consist of poly(L-lactide).

Typically, the poly(L-lactide) has an inherent viscosity midpoint of from 1.0 dl/g to 2.5 dl/g. The poly(L-lactide) may for instance have an inherent viscosity midpoint of from 1.5 dl/g to 2.1 dl/g, for instance an inherent viscosity midpoint of about 1.8 dl/g.

The poly(L-lactide) typically has a weight average molecular weight (M_{w}) of from 50,000 g/mol to 250,000 g/mol. Preferably, the poly(L-lactide) has a M_{w} of from 120,000 g/mol to 180,000 g/mol, for instance about 150,000 g/mol.

Any suitable solvent may be employed in the nanofibre precursor solution. A wide range of solvents can be used in electrospinning, including for instance water and polar, non-polar, protic and aprotic organic solvents. The solvent is chosen to suit the polymer or blend employed, particularly so that a homogeneous solution of the required concentration of the polymer can be obtained.

HFIP is especially suitable when the polymer is poly(L-lactide).

Typically, the solvent is 1,1,1,3,3,3-Hexafluoroisopropanol (HFIP).

The concentration of the polymer in the solution should be high enough to achieve adequate chain entanglements in order for a continuous fibre to be formed. Typically, the concentration of the polymer in said solvent is from about 5 wt% to about 20 wt%. The concentration of the polymer in said solvent may for instance be from about 8 wt% to about 17 wt%. For instance, the concentration of the polymer in said solvent may be about 10wt%, or about 15wt%. The concentration of the polymer in said solvent may for example be from about 5 wt% to about 15 wt%. For instance, the concentration of the polymer in said solvent may be about 10wt%.

The nanofibre precursor solution may be a solution of from 5wt% to 20wt% poly(L-lactide) in an organic solvent, such as HFIP. The nanofibre precursor solution may for instance be a solution of from 5wt% to 15wt% poly(L-lactide) in an organic solvent, such as HFIP. In one embodiment, the nanofibre precursor solution is a solution of from 8 wt% to 12 wt% poly(L-lactide) in an organic solvent, for instance a solution of about 10 wt % poly(L-lactide) in an organic solvent. Alternatively, the nanofibre precursor solution may be a solution of from 13 wt% to 17 wt% poly(L-lactide) in an organic solvent, for instance a solution of about 15 wt % poly(L-lactide) in an organic solvent. The organic solvent is typically HFIP.

Typically, the dispensing capillary of the fibre forming module has an inner diameter of from about 0.5 mm to about 1.0 mm.

In order to ensure uniform deposition on the collection substrate, the electrospinning typically further comprises moving at least a portion of the fibre collection device relative to the fibre forming module during said deposition.

Thus, usually, the electrospinning further comprises moving at least a portion of the fibre collection device during said deposition.

Typically the fibre collection device comprises a rotatable portion. Usually, the electrospinning further comprises rotating the rotatable portion during said deposition. The rotatable portion is typically a rotatable drum. It is typically rotated at a rate of from about 80 rpm to about 120 rpm during the deposition.

Typically, therefore, the electrospinning further comprises rotating at least a portion of the fibre collection device during said deposition. Usually, the rotation is at a rate of from about 80 rpm to about 120 rpm.

Usually, the fibre collection device comprises a rotatable drum and the electrospinning further comprises rotating said drum during said deposition. Typically, said rotation is at a rate of from about 80 rpm to about 120 rpm.

Deposition of the scaffold layer on the collection substrate is continued until a layer of a particular desired thickness has been obtained. Thus, typically the step of feeding said nanofibre precursor solution through the dispensing capillary whilst applying said voltage is performed until the thickness of said scaffold layer is from 10 µm to 150 µm. More typically, the step of feeding said nanofibre precursor solution whilst applying said voltage is performed until the thickness is from 30 µm to 120 µm. For instance, the step of feeding whilst applying said voltage may be performed until the thickness is from about 50 µm to about 100 µm.

Typically, the flow rate at which the nanofibre precursor solution is fed through the dispensing capillary is from 100 µl/hr to 2000 µl/hr. More typically, it is from 400 µl/hr to 700 µl/hr, for instance from 400 µl/hr to 600 µl/hr, for example about 500 µl/hr. In another embodiment, it is from 500 µl/hr to 700 µl/hr, for example about 600 µl/hr.

The distance between the dispensing capillary and the collection substrate is typically from 200 mm to 400 mm. More typically, it is from 250 mm to 350 mm, for instance about 300 mm.

The voltage applied across the dispensing capillary and the fibre collection device is typically from 10 kV to 15 kV. More typically, it is from 12 kV to 13 kV, for instance about 12.5 kV.

Usually, the electrospinning is performed at a temperature of from 22 °C to 28 °C.

More typically, the electrospinning is performed at a temperature of from 23 °C to 27 °C, for instance about 25 °C.

Typically, the electrospinning is performed in air having a relative humidity of from 20 % to 30 %. The electrospinning may for instance be performed in air having a relative humidity of 23 % to 27 %, for instance about 25 %.

A particularly preferred embodiment of the scaffold layer is produced by the electrospinning process defined above when: the polymer is poly(L-lactide) having an inherent viscosity midpoint of from 1.0 dl/g to 2.5 dl/g, preferably from 1.5 dl/g to 2.1 dl/g; the solvent is 1,1,1,3,3,3-Hexafluoroisopropanol; the concentration of the polymer in said solvent is from 5 wt% to 20 wt%; the flow rate at which the nanofibre precursor solution is fed through the dispensing capillary is from 100 µl/hr to 2000 µl/hr; the distance between the dispensing capillary and the collection substrate is from 200 mm to 400 mm; the voltage applied across the dispensing capillary and the fibre collection device is from 10 kV to 15 kV; the electrospinning is performed at a temperature of from 22 °C to 28 °C; and the electrospinning is performed in air having a relative humidity of from 20 % to 30 %.

Some embodiments of the scaffold layer are produced by the electrospinning process defined above when: the polymer is poly(L-lactide) having an inherent viscosity midpoint of from 1.0 dl/g to 2.5 dl/g, preferably from 1.5 dl/g to 2.1 dl/g; the solvent is 1,1,1,3,3,3-Hexafluoroisopropanol; the concentration of the polymer in said solvent is from 5 wt% to 15 wt%; the flow rate at which the nanofibre precursor solution is fed through the dispensing capillary is from 100 µl/hr to 2000 µl/hr; the distance between the dispensing capillary and the collection substrate is from 200 mm to 400 mm; the voltage applied across the dispensing capillary and the fibre collection device is from 10 kV to 15 kV; the electrospinning is performed at a temperature of from 22 °C to 28 °C; and the electrospinning is performed in air having a relative humidity of from 20 % to 30 %.

A further particularly preferred embodiment of the scaffold layer may be produced by the electrospinning process defined above when: the polymer is poly(L-lactide) having an inherent viscosity midpoint of from 1.5 dl/g to 2.1 dl/g; the solvent is 1,1,1,3,3,3-Hexafluoroisopropanol; the concentration of the polymer in said solvent is from 8 wt% to 17 wt%; the flow rate at which the nanofibre precursor solution is fed through the dispensing capillary is from 450 µl/hr to 650 µl/hr; the distance between the dispensing capillary and the collection substrate is from 250 mm to 350 mm; the voltage applied across the dispensing capillary and the fibre collection device is from 12 kV to 13 kV; the electrospinning is performed at a temperature of from 23 °C to 27 °C; and the electrospinning is performed in air having a relative humidity of from 23 % to 27 %.

Some embodiments of the scaffold layer may be produced by the electrospinning process defined above when: the polymer is poly(L-lactide) having an inherent viscosity midpoint of from 1.5 dl/g to 2.1 dl/g; the solvent is 1,1,1,3,3,3-Hexafluoroisopropanol; the concentration of the polymer in said solvent is from 8 wt% to 12 wt%; the flow rate at which the nanofibre precursor solution is fed through the dispensing capillary is from 450 µl/hr to 550 µl/hr; the distance between the dispensing capillary and the collection substrate is from 250 mm to 350 mm; the voltage applied across the dispensing capillary and the fibre collection device is from 12 kV to 13 kV; the electrospinning is performed at a temperature of from 23 °C to 27 °C; and the electrospinning is performed in air having a relative humidity of from 23 % to 27 %.

The electrospinning process may further comprise: drying the scaffold layer thus produced. Typically, the scaffold layer is dried under vacuum.

Typically, the electrospinning process further comprises: removing the scaffold layer thus produced from the collection substrate and disposing the scaffold layer on the plate base. The collection substrate typically comprises aluminium foil or a silicone-coated sheet.

As mentioned above, the scaffold layer usually matches the shape of the array of sample wells in the plate frame. In particular, the scaffold layer is typically rectangular, to match the typical shape of the array of sample wells in the plate frame.

Usually therefore, the electrospinning process further comprises removing the scaffold layer thus produced from the collection substrate, cutting the scaffold layer to fit the plate frame and/or plate base, and disposing said scaffold layer on the plate base.

The electrospinning process may for instance further comprise removing the scaffold layer thus produced from the collection substrate, cutting the scaffold layer to a size of at least about 105 mm in length and at least about 69 mm in width, and disposing said scaffold layer on the plate base. Usually, the scaffold layer is cut to a size of at least about 109 mm in length and at least about 73 mm in width. In a preferred embodiment the scaffold layer is cut to a size of from 105 to 113 mm in length and from 69 to 77 mm in width. More typically, the scaffold layer is cut to a length of about 109 mm and to a width of about 73 mm. Usually, the scaffold layer is cut into a rectangular shape. Thus, the scaffold layer may be cut into a rectangular shape having a length of from 105 to 113 mm and a width of from 69 to 77 mm, or for instance into a rectangular shape having a length of about 109 mm and a width of about 73 mm.

The present invention is further illustrated in the Examples which follow:

### EXAMPLES

### Example 1 - Scaffold fabrication and preparation of multi-well plate

### 1. Scaffold fabrication by electrospinning

A solution was prepared by dissolving 3.0 grams of polymer, poly(L-lactide), (poly[(3S-cis)-3,6-dimethyl-1,4-dioxane-2,5-dione]), having an Inherent Viscosity Midpoint of 1.8 dl/g, in 27.0 grams of the solvent, 1,1,1,3,3,3-Hexafluoroisopropanol (HFIP), with continuous stirring at room temperature until a homogeneous solution of the required concentration, 10 wt%, was obtained. The polymer solution was loaded into a plastic syringe connected to a metal capillary (21 gauge) via PTFE tubing. The metal capillary was connected to the positive terminal of a high voltage, DC power supply, at a fixed distance of 300 mm from an earthed metallic collection device (covered in an Aluminium foil/silicone coated release paper substrate) onto which the fibres were deposited. The collection device was a rotating drum to ensure uniform deposition of the material. Fibres were produced by passing the polymer solution at a flow rate of 500 µl/hr through the metal capillary whilst applying a voltage of 12.5 kV to the capillary in order to establish an electric field between the capillary and the collection device. The applied voltage was sufficiently high enough to overcome the surface tension of the polymer droplet at the tip of the capillary, and form a Taylor Cone from the droplet, from which a continuous fibre of the polymer was extracted. As fibre travelled towards the grounded collector, the solvent rapidly evaporated and the fibre became further elongated due to instabilities arising from the coulomic repulsions of the surface charges on the jet. The instabilities in the jet resulting from the high charge density caused the jet to whip about rapidly resulting in a nano/micro diameter solid (dry) filament of poly(L-lactide) with an average fibre diameter of 2 µm. The collector was rotated slowly (at a rate of around 100 rpm) resulting in the deposition of a non-woven fibrous membrane on the substrate. After a fixed amount of material was deposited to generate a membrane of a certain thickness, typically 50 µm or 100 µm, the membrane was dried under vacuum for 24-48 hours at room temperature (approx. 25°C) in order to remove any residual solvent/moisture from the fibres. The electrospinning process was performed at a temperature of 25°C and a relative humidity of 25%.

The scaffold membranes produced by the process had the following properties:
- Average fibre diameter = 2 µm *
- Diameter distribution = %RSD (relative standard deviation) <10% *
- Membrane thickness = 50 µm (first membrane), 100 µm (second membrane)
- Dimensions = 109 x 73 mm
- Morphology = Random, non-woven fibre membrane

Automated image characterisation is generally performed using a Phenom Fibremetric SEM system (Lambda Photometrics), which enables the automated analysis of multiple images in order to determine the average fibre diameter and the relative standard deviation. The Fibremetric software automatically identifies the location of the fibres due to the contrast within the captured SEM image and measures the diameter of each fibre 20 times at a specific location. Typically around 100 of such measurements are performed per image.

Alternatively, the diameter of the fibres can be obtained via manual measurements/analysis of multiple SEM images.

* The average fibre diameter and diameter distribution values above were obtained via manual measurements/analysis of multiple SEM images.

### 2. Preparation of multi-well plate

A vacuum dried electrospun membrane (scaffold layer) produced in 1 was cut into a rectangle of length 109 mm and width 73 mm, which dimensions were determined by the area of the multi-well plate frame onto which it was fixed. The membrane was removed from the backing substrate (the aluminium foil/silicone release paper from the electrospinning process), and placed top-side down onto the base of a black multi-well plate frame. The steps of this process are illustrated schematically in Fig. 1. A 170 µm thick clear polystyrene substrate (plate base) was then applied to sandwich the scaffold membrane between the plate base and the black plate frame. A 150 µm thick clear polystyrene protective sheet was then applied. The plate was then inserted into a laser welding machine and clamped to a pre-aligned contact mask (either a copper/quartz mask or chrome/borosilicate mask). The mask was designed to prevent light reaching the areas of scaffold membrane where the wells are plus 200-300 microns around the edges to prevent damaging the scaffold fibres around the edge of the individual wells. The laser welding was performed by scanning a diode laser @ wavelength of 940 nm across top of mask at a scan rate of 200 mm/min, in order to melt the black frame (the scaffold membrane is also melted) to form a weld with the clear substrate in the unmasked regions. The welded multi-well plate was then removed from the clamp, and the 150 µm clear polystyrene sheet was then removed from the plate base to give the final product, in which the 170 µm substrate (plate base) was welded to the black frame to form water tight seals around the individual wells containing unaltered fibrous membrane.

### Example 2 - Measurement of fibre diameter and diameter distribution

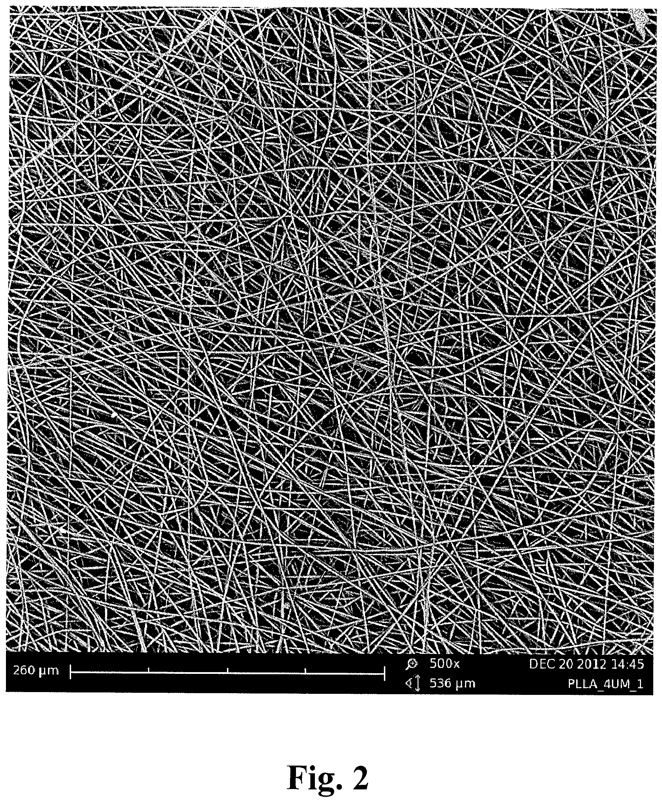

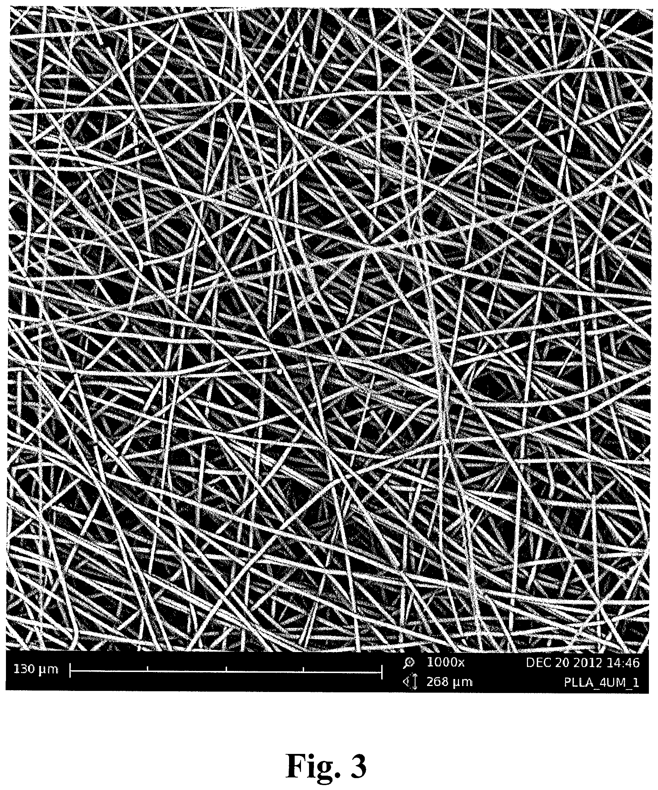

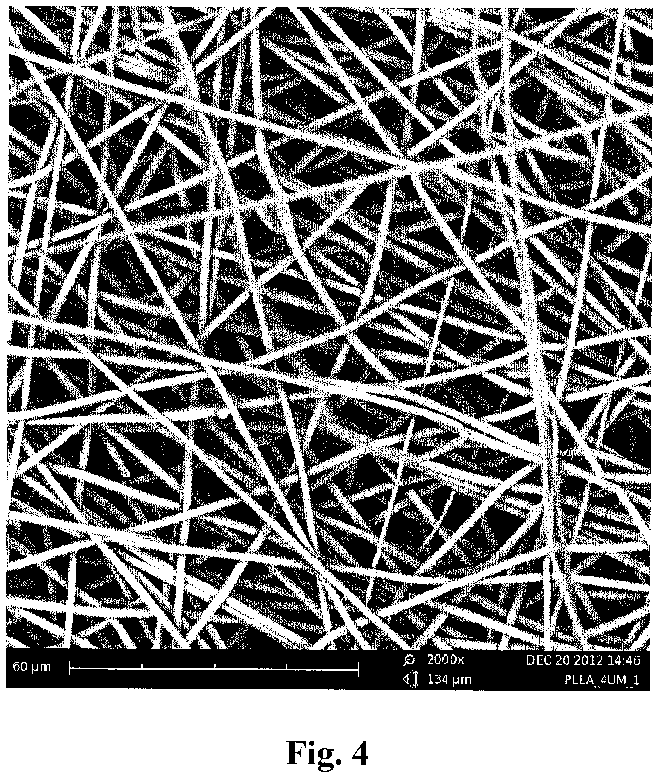

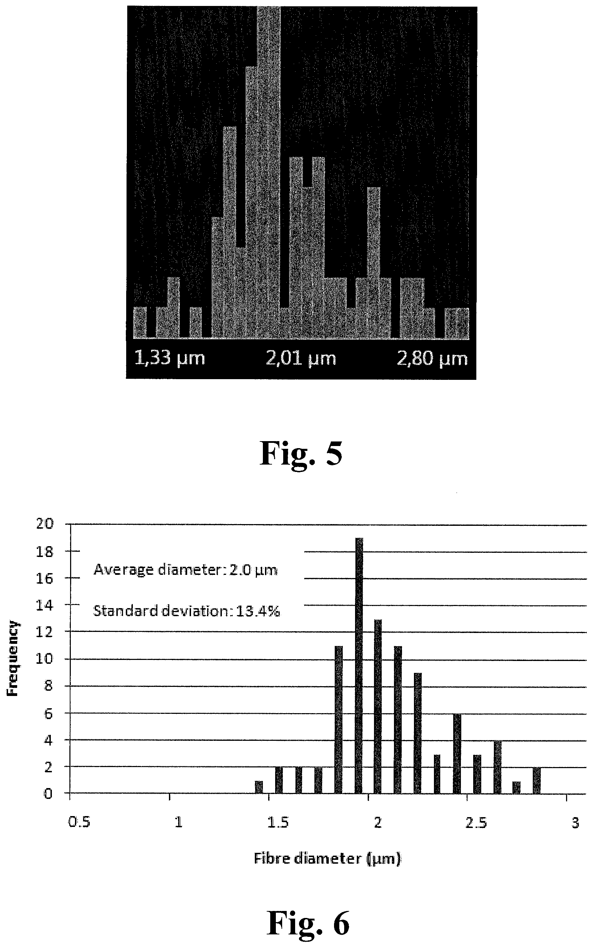

Scaffolds were fabricated by electrospinning using the method described in Example 1. The electrospun samples were imaged using a Phenom G2 pro scanning electron microscope, which allows for automatic fibre diameter measurements by the Fibremetric Software Application from PhenomWorld. Fibre samples were mounted onto conductive adhesive tabs and loaded into a charge reduction sample holder, which was subsequently inserted into the scanning electron microscope (SEM). SEM images of the fibres were obtained, examples of which are shown in Figure 2 (500x magnification image), Figure 3 (1000x magnification image) and Figure 4 (2000x magnification image). To obtain a statistically sound measurement of the fibre diameter distribution, the diameters of 100 fibres were measured using an image at 1000-2000x magnification. In some instances, the software recognises two fibres which cross or stick together as one; these data points were deleted. A histogram of the diameter distribution, generated by the PhenomWorld software, is shown in Figure 5. The data (a list of all fibre diameters, plus various other parameters) were exported into an XML file, which was then loaded into Excel. The mean diameter and standard deviation of the diameter distribution was obtained using the Excel formulae "AVERAGE" and "STDEV". The mean fibre diameter for the scaffold fabricated using the method described in Example 1 was found to be 2.0 µm. The standard deviation of the fibre diameter distribution was found to be 13.4%. A chart of the diameter distribution (Figure 6) was generated by counting the number of fibres between the highest and lowest value at intervals of 0.1 µm and plotting the data in Excel as a column chart.

A standard operating procedure for analysing the diameter of electrospun fibres as described above is presented below.

### Standard Operating Procedure: Diameter Analysis of Electrospun Fibres

### Purpose

To provide instructions for the statistical analysis of the diameter distribution of electrospun fibres based using a Phenom G2 pro scanning electron microscope and the Fibermetric software application from PhenomWorld.

### Scope

This protocol covers the use of the Phenom G2 pro scanning electron microscope, automatic measurement of fibre diameters with the Fibermetric software application, and statistical analysis of the obtained data using Microsoft Excel.

### Equipment needed

### • Phenom G2 pro scanning electron microscope

### Software needed:

- Fibermetric software application
- Microsoft Excel

### Procedure

- Cut out a small piece of fibre (ca. 0.5 cm², either with or without aluminium foil) and stick it onto a stub using a conductive sticky pad. Place the stub into the Phenom G2 pro sample holder and rotate upper part of the sample holder until the surfaces of the sample and sample holder are in the same plane. Then move sample further down by rotating the upper part of the sample holder by 5 more notches.
- Insert sample holder into the Phenom G2 pro and close lid. The sample will be automatically loaded.
- Switch from optical mode into SEM mode, autofocus on the sample surface, and adjust contrast and brightness.
- Take one image at 500x magnification, one at 1000x magnification, and one at 2000x magnification. If the fibres are small (<1 um in diameter), a fourth image at 5000x magnification may be taken, although this can cause the fibres to melt in some cases.
- Once the 2000x image has been taken, open the Fibermetric Software Application from PhenomWorld on the computer next to the SEM computer. Click on "Acquire image from Phenom" to load image into the program. Alternatively, open an old project or image file.
- Click on the arrow button (>) for the next step. Click on "process" to measure the diameter of 100 fibres. This will take app. 30-60 s. In some instances, the software recognises two fibres which cross or stick together as one; these data points should be manually deleted.
- Click on the arrow button (>) for the next step. Choose filename and folder, then click on "Save Project" and "Generate Report", which will save the raw data (a list of all fibre diameters, plus various other parameters) as an XML file.
- Import XML file into Excel. Calculate mean diameter and standard deviation of the diameter distribution by using the formulae "AVERAGE" and "STDEV". Generate chart of the diameter distribution by counting the number of fibres between the highest and lowest value at intervals of 0.1 um using the "FREQUENCY" formula and plot the obtained data as a column chart.

### References

www.phenom-world.com

### Example 3 - Comparative testing of scaffolds of 2µm fibre diameter and 4µm fibre diameter, each in two (2) scaffold thicknesses

### Production of scaffolds with 2µm fibre diameter

Scaffold membranes having thicknesses of 50 µm and 100 µm, and containing poly(L-lactide) fibres with a mean diameter of 2µm, were produced by the electrospinning method described in Example 1 above.

### Production of scaffolds with 4µm fibre diameter

Scaffold membranes having thicknesses of 50 µm and 100 µm, and containing poly(L-lactide) fibres with a mean diameter of 4µm, were produced by the following method:
A solution was prepared by dissolving 4.5 grams of polymer, poly(L-lactide), (poly[(3S-cis)-3,6-dimethyl-1,4-dioxane-2,5-dione]), having an Inherent Viscosity Midpoint of 1.8 dl/g, in 25.5 grams of the solvent, 1,1,1,3,3,3-Hexafluoroisopropanol (HFIP), with continuous stirring at room temperature until a homogeneous solution of the required concentration, 15 wt%, was obtained. The polymer solution was loaded into a plastic syringe connected to a metal capillary (21 gauge) via PTFE tubing. The metal capillary was connected to the positive terminal of a high voltage, DC power supply, at a fixed distance of 300 mm from an earthed metallic collection device (covered in an Aluminium foil/silicone coated release paper substrate) onto which the fibres were deposited. The collection device was a rotating drum to ensure uniform deposition of the material. Fibres were produced by passing the polymer solution at a flow rate of 600 µl/hr through the metal capillary whilst applying a voltage of 12.5 kV to the capillary in order to establish an electric field between the capillary and the collection device. The applied voltage was sufficiently high enough to overcome the surface tension of the polymer droplet at the tip of the capillary, and form a Taylor Cone from the droplet, from which a continuous fibre of the polymer was extracted. As fibre travelled towards the grounded collector, the solvent rapidly evaporated and the fibre became further elongated due to instabilities arising from the coulomic repulsions of the surface charges on the jet. The instabilities in the jet resulting from the high charge density caused the jet to whip about rapidly resulting in a nano/micro diameter solid (dry) filament of poly(L-lactide) with an average fibre diameter of 4 µm. The collector was rotated slowly (at a rate of around 100 rpm) resulting in the deposition of a non-woven fibrous membrane on the substrate. After a fixed amount of material was deposited to generate a membrane of a certain thickness, typically 50 µm or 100 µm, the membrane was dried under vacuum for 24-48 hours at room temperature (approx. 25°C) in order to remove any residual solvent/moisture from the fibres. The electrospinning process was performed at a temperature of 25°C and a relative humidity of 25%.

The scaffold membranes produced by the process had the following properties:
- Average fibre diameter = 4 µm **
- Diameter distribution = %RSD (relative standard deviation) < 15 % **
- Membrane thickness = 50 µm (first membrane), 100 µm (second membrane)
- Morphology = Random, non-woven fibre membrane
** The average fibre diameter and diameter distribution values above were obtained by the automated method described in Example 2.

### Scaffold Evaluation: cell attachment - scaffold loading efficiency

Experiments to measure the loading efficiency of cells on scaffolds of the invention were performed using the following cell type: human breast cancer cell HMT3909S8.

Exploratory experiments were carried out in which the scaffolds were seeded with the cells and allowed to culture for 24 hours. The scaffold cell number was measured after 24 hours of culture and the cell loading efficiency was determined. Confirmatory experiments were also performed, which were the same as the exploratory experiments except that cell culture was continued up to 10 days to monitor cell growth within the scaffold.

### Experimental design

### 1. Exploratory Experiment

The Exploratory Experiment made direct measurements of cell number seeded into scaffolds and of cell number associated with scaffolds after 24 h of cell culture. These measurements were facilitated by the transfer of scaffolds between wells; that is, moving of scaffolds containing cells from the original well in which cells were seeded to a new (clean) well in which attached cell number was then measured. This allowed scaffold-associated cells to be distinguished from cells that did not attach to the scaffolds and therefore remained in the original well after scaffold transfer to a clean well.

The experiment was performed using the HMT3909S8 breast cancer cell line. Cell seeding into scaffolds was performed manually, by addition of cell suspension harvested from a standard 2D culture. The experiment used a scaffold loading of 50,000 cells, which was added in a total volume of 1 ml of culture medium. The culture medium was CDM3, a proprietary fully-defined culture medium formulated for use with the HMT3909S8 cell line.

The number of cells present inside scaffolds (or not attached to scaffolds and remaining in the original well) was measured as DNA content, measured directly after complete lysis of resident cells to release their contents into the culture medium, at which point the scaffold was removed to avoid assay interference. The assay uses DNA binding of a fluorescent dye, to generate a sensitive assay readout.

### 2. Confirmatory Experiment

The exploratory experiment established scaffold seeding conditions required for performance of a Confirmatory Experiment, in which cell growth within scaffolds was monitored for a period of up to 10 days. Unless contra-indicated by the Exploratory Experiment, scaffold loading with cells for the Confirmatory Experiment was the same as that for the Exploratory Experiment. The principal features of the Confirmatory Experiment were as follows:
- Seeding of 50,000 cells into scaffold variants contained in wells of 12-well culture plates, or into empty wells for comparative 2D culture; quadruplicate tests were performed with treatments measuring cell number ("CN scaffolds")
- Immediately after seeding, culture the cells at 37°C and 5% (v/v) CO2 in air
- At 24 h, removal of all scaffolds into clean wells, and performance of a DNA assay in the original well, to measure the number of cells left behind i.e. not associated with the scaffold
- After transfer to fresh wells; for half of the wells containing CN scaffolds, cell lysis and equilibration of lysate with culture medium, followed by scaffold removal and assay of DNA, to measure the number of cells associated with the scaffold at 24 h; a set of DNA standards was run to allow direct calculation of cell DNA content
- With the remaining wells containing CN scaffolds, culture for a further 10 days, with medium replacement on the fourth, sixth and eighth day of this extended culture period; also, at day 4, transfer of scaffolds to a clean well, with performance of a DNA assay in the original well, to measure the number of cells left behind i.e. not associated with the scaffold four days after placement in the well.
- After a further 10 days of culture, lysis of cells in wells containing CN scaffolds, lysate equilibration with culture medium, followed by scaffold removal and DNA assay; a set of DNA standards was run to allow direct calculation of DNA content; equivalent measurements were performed on cells cultured in parallel without scaffolds.

### Results

Figures 7 and 8 show the results of the exploratory and confirmatory experiments. Figure 7 is a bar chart showing the % cell loading efficiency (y axis) observed at 24 hours post seeding, in (i) the exploratory experiments, and (ii) the confirmatory experiments, on each of the following scaffold types: (a) 50 µm scaffold thickness, average fibre diameter of 2 µm; (b) 50 µm scaffold thickness, average fibre diameter of 4 µm; (c) 100 µm scaffold thickness, average fibre diameter of 2 µm; and (d) 100 µm scaffold thickness, average fibre diameter of 4 µm. The numbers shown in parentheses adjacent the error bars in Figure 7 are the coefficients of variance (% CV). The % CV values are low, showing that for each of the scaffolds of the invention, cell culture growth and cell loading efficiency was remarkably consistent and reproducible.

Cell seeding efficiency was found to differ with scaffold fibre diameter. In both the exploratory and confirmatory experiments, the loading efficiency of this particular cell type increased with greater scaffold fibre diameter and scaffold thickness. Thus, the lowest seeding density was observed with the 50µm scaffold made with 2µm fibre, and highest with the 100µm scaffold made with 4µm fibre.

Figure 8 is a bar chart showing the scaffold cell number (y axis) for scaffolds of the invention (i) as initially seeded, (ii) 24 hours after seeding, and (iii) 10 days after seeding, for each of the following scaffold types: (a) 50 µm scaffold thickness, average fibre diameter of 2 µm; (b) 50 µm scaffold thickness, average fibre diameter of 4 µm; (c) 100 µm scaffold thickness, average fibre diameter of 2 µm; and (d) 100 µm scaffold thickness, average fibre diameter of 4 µm. Again, the low %CV values (shown in parentheses adjacent the error bars) are indicative of consistent and reproducible cell culture growth in the scaffolds of the invention.

The cell number after 10 days ranged from a 3.8-fold increase (in the 100 µm thick, 4 µm diameter fibre scaffolds) to a 6.5-fold increase (in the 50 µm thick, 2 µm fibre diameter scaffolds). The 50 µm-thick scaffolds produced from 2 µm- or 4 µm-diameter fibre contained similar numbers of cells after 10 days' growth. However, as seeding efficiency into the 2 µm-fibre scaffold was lower, the fold growth in this scaffold variant was greater than for the 4 µm-fibre version (6.5- vs 5.2-fold). A different situation was observed with the 100 µm-thick scaffolds, where the 2 µm-diameter fibre supported significantly more cell growth, such that cell number after 10 days was higher, even though seeding efficiency had been somewhat lower.

### Scaffold Evaluation: induction of apoptosis experiments

Experiments were then performed (using the same cell type, human breast cancer cell HMT3909S8) to compare staurosporine-induced apoptosis in (i) conventional 2D cell culture (positive control), and (ii) cells cultured in 3D using the electrospun scaffolds of the invention in the following four formats: (a) 50 µm scaffold thickness, average fibre diameter of 2 µm; (b) 50 µm scaffold thickness, average fibre diameter of 4 µm; (c) 100 µm scaffold thickness, average fibre diameter of 2 µm; and (d) 100 µm scaffold thickness, average fibre diameter of 4 µm.

### Experimental design (continued from the experimental design section above relating to the Confirmatory Experiment)

After transfer to fresh wells, scaffolds were cultured for a further 10 days, with medium change and scaffold transfer as for CN scaffolds; after 10 days, medium replacement and addition of apoptosis positive control staurosporine, then culture for a further 24 h. On the final day of the experiment, apoptosis was assayed as cleavage of a peptide substrate recognised by the executor caspase 3/7, a late-stage event in the process of cell death by apoptosis. Substrate cleavage produced a fluorescent signal measurable in a standard plate reader.

### Results

The results are shown in Figure 9. Figure 9 is a bar chart showing induced versus basal apoptosis (fold induction) on the y axis, for (i) a conventional 2D cell culture (positive control), and (ii) the following formats of scaffolds of the invention: (a) 50 µm scaffold thickness, average fibre diameter of 2 µm; (b) 50 µm scaffold thickness, average fibre diameter of 4 µm; (c) 100 µm scaffold thickness, average fibre diameter of 2 µm; and (d) 100 µm scaffold thickness, average fibre diameter of 4 µm.

The Figure shows that the positive control, staurosporine, produced the expected stimulation of apoptosis in the 2D culture. Cells in a 3D scaffold environment showed an attenuated induction of apoptosis, ranging from 1.9-fold (for the 100 µm / 2 µm fibre scaffold) to 3.5-fold (for the 50 µm / 4 µm scaffold). It was notable that, for both the 50 µm-thick scaffolds and the 100 µm-thick scaffolds, apoptosis induction was lower in scaffolds formed from 2 µm fibre than those made from 4 µm scaffolds.

The apoptosis data present a positive picture of cell performance with the scaffolds of the invention. Growing in a 3D environment consistently protected the cells from apoptosis induction by staurosporine, a key determinant of the value of the scaffold culture environment. It was noteworthy that this protective effect varied between the architectures tested, with the 2 µm fibre-generated structures appearing to offer greater protection with both scaffold thicknesses.

Scaffolds produced from 2 µm-diameter fibre showed lower (50 µm-thick scaffold) or similar (100 µm-thick scaffold) cell attachment compared with the 4 µm-diameter fibre scaffolds. There appears to be little to choose between the 50 µm-thick and 100 µm-thick in respect of cell growth and apoptosis susceptibility, but other factors (cost, microscopic accessibility) may tend to favour the 50 µm-thick scaffold format.

## Claims

1. A multi-well assay plate, comprising:
a transparent plate base, which defines the bottom of a plurality of sample wells;
a scaffold layer disposed on the plate base, which scaffold layer provides a porous three-dimensional network of electrospun polymer nanofibres in each of said sample wells, wherein the thickness of the scaffold layer in each of said sample wells is from 10 µm to 150 µm and the mean diameter of the polymer nanofibres is from 500 nm to 10 µm; and
a plate frame, which defines the side walls of said sample wells;
wherein the plate frame is opaque and is welded to the plate base through the scaffold layer.

2. A multi-well assay plate according to claim 1 wherein the mean diameter of the polymer nanofibres is from 1 µm to 5 µm.

3. A multi-well assay plate according to claim 1 or claim 2 wherein the relative standard deviation from said mean is less than or equal to 15%.

4. A multi-well assay plate according to any one of claims 1 to 3 wherein the polymer nanofibres comprise poly(L-lactide); poly(glycolic acid); polyhydroxybutyrate; polystyrene; polyethylene; polypropylene; poly(ethylene oxide); a poly(ester urethane); poly(vinyl alcohol); polyacrylonitrile; polylactide; polyglycolide; polyurethane; polycarbonate; polyimide; polyamide; aliphatic polyamide; aromatic polyamide; polybenzimidazole; poly(ethylene terephthalate); poly [ethyl ene-co-(vinyl acetate)]; poly(vinyl chloride); poly(methyl methacrylate); poly(vinyl butyral); poly(vinylidene fluoride); poly(vinylidene fluoride-co-hexafluoropropylene); cellulose acetate; poly(vinyl acetate); poly(acrylic acid); poly(methacrylic acid); polyacrylamide; polyvinylpyrrolidone; poly(phenylene sulfide); hydroxypropylcellulose; polyvinylidene chloride; polytetrafluoroethylene; a polyacrylate; a polymethacrylate; a polyester; a polysulfone; a polyolefin; polysilsesquioxane; silicone; epoxy; cyanate ester; a bis-maleimide polymer; polyketone; polyether; polyamine; polyphosphazene; polysulfide; an organic/inorganic hybrid polymer thereof, a copolymer thereof or a blend thereof; poly(lactide-co-glycolide); polylactide-co-poly(ε-caprolactone); poly(L-lactide)-co-poly(ε-caprolactone); a blend of poly(vinyl alcohol) and poly(acrylic acid); collagen; a blend of collagen and poly(ethylene oxide); a blend of collagen and poly(ε-caprolactone); a blend of collagen and polylactide-co-poly(ε-caprolactone); gelatin; a blend of gelatine and poly(ε-caprolactone); a blend of gelatine and poly(ethylene oxide); a blend of casein and poly(vinyl alcohol); a blend of casein and poly(ethylene oxide); lipase; a blend of cellulose and poly(vinyl alcohol); a blend of bovine serum albumin and poly(vinyl alcohol); a blend of luciferase and poly(vinyl alcohol); α-chymotrypsin; fibrinogen; silk; regenerated silk; regenerated *Bombyx mori* silk; a blend of Bombyx mori silk and poly(ethylene oxide); silk fibroin; a blend of silk fibroin and chitosan; a blend of silk fibroin and chitin; a blend of silk and poly(ethylene oxide); artificial spider silk; chitin; chitosan; a blend of chitosan and poly(ethylene oxide); a blend of chitosan and poly(vinyl alcohol); a blend of quaternized chitosan and poly(vinyl alcohol); a blend of hexanoylchitosan and polylactide; cellulose; cellulose acetate; a polyvinylpyrrolidone/polylactide blend; a polyaniline/polystyrene blend; a polyaniline/poly(ethylene oxide) blend; a poly(vinyl chloride)/polyurethane blend; a poly[(m-phenylene vinylene)-co-(2,5-dioctyloxy-p-phenylene vinylene)]/poly(ethylene oxide) blend; a poly[2-methoxy-5-(2'-ethylhexyloxy)-1,4-phenylene vinylene] (MEH-PPV)/polystyrene blend; a polyaniline/polystyrene blend; a polyaniline/polycarbonate blend; a poly(ethylene terephthalate)/ poly(ethylene terephthalate)-co-poly(ethylene isophthalate) blend; a polysulfone/polyurethane blend; a chitosan/polylactide blend; a polyglycolide/chitin blend; a polylactide/poly(lactide-co-glycolide) blend; polylactide-b-poly(ethylene oxide) block copolymer; poly(lactide-co-glycolide)-b-poly(ethylene oxide) block copolymer; poly[(trimethylene carbonate)-b-(ε-caprolactone)] block copolymer; polystyrene-b-polydimethylsiloxane block copolymer; polystyrene-b-polypropylene block copolymer; polystyrene-b-polybutadiene-b-polystyrene block copolymer; polystyrene-b-polyisoprene block copolymer; a blend of montmorillonite with polyamide 6, polyamide 6,6, poly(vinyl alcohol), poly(methyl methacrylate) or polyurethane as the carrier material; a blend of a polymer carrier and noble metal nanoparticles; a blend of poly(acrylonitrile)-co-poly(acrylic acid) and Pd nanoparticles; a blend of poly(ethylene oxide) and Au nanoparticles; a blend of polyvinylpyrrolidone and Ag nanoparticles; a blend of poly(acrylonitrile) and Ag nanoparticles; a blend of a polymer carrier and magnetic nanoparticles; a blend of Fe₃O₄ nanoparticles with poly(ethylene oxide) or poly(vinyl alcohol); a blend of poly(ε-caprolactone) and FePt nanoparticles; a blend of polyurethane and MnZnNi nanoparticles; a blend of poly(methyl methacrylate) and Co nanoparticles; a blend of a polymer and carbon nanotubes; carbon nanotubes blended with poly(acrylonitrile), poly(ethylene oxide), poly(vinyl alcohol), polylactide, polycarbonate, polystyrene, polyurethane or poly(methyl methacrylate); a blend of a polymer and a metal oxide or metal sulphide; a blend of polymer and TiO₂; a blend of a TiO₂ and a polymer selected from polyvinylpyrrolidone, poly(vinyl acetate) and poly(acrylonitrile); a blend of polymer and ZrO₂; a blend of ZrO₂ and a polymer selected from polyvinylpyrrolidone, poly(vinyl acetate) and poly(vinyl alcohol); or a blend of a polymer with any of ZnO, CuO, NiO, CeO₂, Mn₃O₄, Mn₂O₃/Mn₃O₄, MoO₃, BaTiO₃, Y₂O₃, Gd₂O₃, Ta₂O₅, Co₃O₄, Ba_{0.6}Sr_{0.4}TiO₃, SiO₂, CdS, PbS and Ag₂S.

5. A multi-well assay plate according to any one of the preceding claims wherein the polymer nanofibres comprise poly(L-lactide).

6. A multi-well assay plate according to any one of the preceding claims wherein the multi-well plate is:
a 96-well plate wherein the sample wells are arranged in an array of 12 x 8 wells;
a 384-well plate wherein the sample wells are arranged in an array of 24 x 16 wells;
a 1536-well plate wherein the sample wells are arranged in an array of 48 x 32 wells; or
a 3456-well plate wherein the sample wells are arranged in an array of 72 x 48 wells.

7. A process for producing a multi-well assay plate, which multi-well assay plate comprises:
a transparent plate base, which defines the bottom of a plurality of sample wells;
a scaffold layer disposed on the plate base, which scaffold layer provides a porous three-dimensional network of electrospun polymer nanofibres in each of said sample wells, wherein the thickness of the scaffold layer in each of said sample wells is from 10 µm to 150 µm and the mean diameter of the polymer nanofibres is from 500 nm to 10 µm; and
a plate frame, which defines the side walls of said sample wells;
wherein the plate frame is opaque and is welded to the plate base through the scaffold layer;
which process comprises:
(a) disposing a scaffold layer between an opaque plate frame and a transparent plate base, wherein the plate frame defines side walls for a plurality of sample wells, the plate base defines a bottom for said plurality of sample wells, and the scaffold layer comprises a porous three-dimensional network of electrospun polymer nanofibres, wherein the thickness of the scaffold layer is from 10 µm to 150 µm and the mean diameter of the polymer nanofibres is from 500 nm to 10 µm; and
(b) welding the plate frame to the plate base, through the scaffold layer, by laser welding or by ultrasonic welding, to form a watertight or hermetic seal between the plate frame and the plate base.

8. A process according to claim 7 wherein said welding (b) comprises:
heating the plate frame, by exposing it to a laser or by ultrasonic energy, and thereby melting the plate frame at the point of contact between the plate frame and the scaffold layer; and
allowing the melt to cool to form a weld between the plate frame and the plate base.

9. A process according to claim 8 wherein said heating melts both the plate frame and the scaffold layer at the point of contact between the plate frame and the scaffold layer, and wherein allowing the melt to cool forms a weld between the plate frame and plate base.

10. A process according to any one of claims 7 to 9 wherein the process comprises
(a1) contacting a scaffold layer with an opaque plate frame and a transparent plate base, wherein the scaffold layer is disposed between the plate frame and plate base, wherein the plate frame defines side walls for a plurality of sample wells, the plate base defines a bottom for said plurality of sample wells, and the scaffold layer comprises a porous three-dimensional network of electrospun polymer nanofibres, wherein the thickness of the scaffold layer is from 10 µm to 150 µm and the mean diameter of the polymer nanofibres is from 500 nm to 10 µm;
(a2) applying a patterned photo mask to the outside of the plate base and applying a force to hold the scaffold layer and plate base between the plate frame and the patterned photo mask; and
(b1) exposing the patterned photo mask to light, comprising exposing the patterned mask to a laser, and thereby heating and melting said plate frame at the point of contact between the plate frame and the scaffold layer,
wherein the patterned photo mask substantially prevents said light from reaching areas of the scaffold layer between the side walls, and wherein the plate base is transparent to said light; and
(b2) allowing the melt to cool to form a weld between the plate frame and the plate base through the scaffold layer,
optionally wherein the process further comprises:
(b3) removing the patterned photo mask.

11. A process according to any one of claims 7 to 9 wherein the process comprises:
(a1) contacting a scaffold layer with an opaque plate frame and a transparent plate base, wherein the scaffold layer is disposed between the plate frame and plate base, wherein the plate frame defines side walls for a plurality of sample wells, the plate base defines a bottom for said plurality of sample wells, and the scaffold layer comprises a porous three-dimensional network of electrospun polymer nanofibres, wherein the thickness of the scaffold layer is from 10 µm to 150 µm and the mean diameter of the polymer nanofibres is from 500 nm to 10 µm;
(a2) applying a protective layer to the outside of the plate base;
(a3) applying a patterned photo mask to the outside of the protective layer and applying a force to hold the scaffold layer, plate base, and protective layer between the plate frame and the patterned photo mask; and
(b1) exposing the patterned photo mask to light, comprising exposing the patterned mask to a laser, and thereby heating and melting said plate frame at the point of contact between the plate frame and the scaffold layer,
wherein the patterned photo mask substantially prevents said light from reaching areas of the scaffold layer disposed between the side walls, and wherein the plate base and the protective layer are transparent to said light; and
(b2) allowing the melt to cool to form a weld between the plate frame and the plate base through the scaffold layer,
optionally wherein the process further comprises:
(b3) removing the patterned photo mask and the protective layer.

12. A process according to claim 10 or claim 11 wherein the laser is an infrared laser, or a near infrared laser.

13. A process according to any one of claims 10 to 12 wherein said laser has a wavelength of from 810 nm to 1064 nm.

14. A process according to any one of claims 10 to 13 wherein the step of exposing the patterned photo mask to a laser comprises scanning a laser beam across the exposed surface of the photo mask.

15. A process according to any one of claims 7 to 14 which further comprises producing the scaffold layer used in step (a) by electrospinning a nanofibre precursor solution onto a collection substrate, wherein the nanofibre precursor solution comprises said polymer dissolved in a solvent.

## Patentansprüche

1. Multiwell-Assayplatte, umfassend:
eine transparente Plattenbasis, die den Boden mehrerer Probenvertiefungen definiert;
eine Gerüstschicht, die auf der Plattenbasis angeordnet ist, wobei die Gerüstschicht ein poröses, dreidimensionales Netzwerk an elektrogesponnenen Polymernanofasern in jeder der Probenvertiefungen bereitstellt, wobei die Dicke der Gerüstschicht in jeder der Probenvertiefungen von 10 µm bis 150 µm beträgt, und wobei der mittlere Durchmesser der Polymernanofasern von 500 nm bis 10 µm beträgt; und
einen Plattenrahmen, der die Seitenwände der Probenvertiefungen definiert;
wobei der Plattenrahmen undurchsichtig und durch die Gerüstschicht auf der Plattenbasis angeschweißt ist.

2. Multiwell-Assayplatte nach Anspruch 1, wobei der mittlere Durchmesser der Polymernanofasern von 1 µm bis 5 µm beträgt.

3. Multiwell-Assayplatte nach Anspruch 1 oder Anspruch 2, wobei relative Standardabweichung von dem Mittel weniger als oder gleich 15 % beträgt.

4. Multiwell-Assayplatte nach einem der Ansprüche 1 bis 3, wobei die Polaymernanofasern Poly(L-lactid); Polyglykolsäure; Polyhydroxybutyrat; Polystyrol; Polyethylen; Polypropylen; Polyethylenoxid; ein Polyesterurethan; Polyvinylalkohol; Polyacrylnitril; Polylactid; Polyglykolid; Polyurethan; Polycarbonat; Polyimid; Polyamid; aliphatisches Polyamid; aromatisches Polyamid; Polybenzimidazol; Polyethylenterephthalat; Poly[ethylen-co-(vinylacetat)]; Polyvinylchlorid; Polymethylmethacrylat; Polyvinylbutyral; Polyvinylidenfluorid; Polyvinylidenfluorid-co-hexafluorpropylen; Celluloseacetat; Polyvinylacetat; Polyacrylsäure; Polymethacrylsäure; Polyacrylamid; Polyvinylpyrrolidon; Polyphenylensulfid; Hydroxypropylcellulose; Polyvinylidenchlorid; Polytetrafluorethylen; ein Polyacrylat; ein Polymethacrylat; einen Polyester; ein Polysulfon; ein Polyolefin; Polysilsesquioxan; Silikon; Epoxid; Cyanatester; ein bis-Maleimidpolymer; Polyketon; Polyether; Polyamin; Polyphosphazen; Polysulfid; ein organisches/anorganisches Hybridpolymer davon, ein Copolymer davon oder eine Mischung davon; Poly(lactid-co-glykolid); Polylactid-co-poly(B-caprolacton); Poly(L-lactid)-co-poly(8-caprolacton); eine Mischung aus Polyvinylalkohol und Polyacrylsäure Kollagen; eine Mischung aus Kollagen und Poly(ethylenoxid; eine Mischung aus Kollagen und Poly(B-caprolacton); eine Mischung aus Kollagen und Polylactid-co-poly(s-caprolacton); Gelatine; eine Mischung aus Gelatine und Poly(B-caprolacton); eine Mischung aus Gelatine und Polyethylenoxid; eine Mischung aus Gelatine und Polyvinylalkohol; eine Mischung aus Casein und Polyethylenoxid; Lipase; eine Mischung aus Cellulose und Polyvinylalkohol; eine Mischung aus Rinderserumalbumin und Polyvinylalkohol; α-Chymotrypsin; Fibrinogen; Seide; regenerierte Seide; regenerierte Bombyx-mori-Seide; eine Mischung aus Bombyx-mori-Seide und Polyethylenoxid; Seidenfibroin; eine Mischung aus Seidenfibroin und Chitosan; eine Mischung aus Seidenfibroin und Chitin; eine Mischung aus Seide und Polyethylenoxid; synthetische Spinnenseide; Chitin; Chitosan; eine Mischung aus Chitosan und Polyethylenoxid; eine Mischung aus Chitosan und Polyvinylalkohol; eine Mischung aus quaternisiertem Chitosan und Polyvinylalkohol; eine Mischung aus Hexanoylchitosan und Polylactid; Cellulose; Celluloseacetat; eine Polyvinylpyrrolidon/Polylactid-Mischung; eine Polyanilin/Polystyrol-Mischung; eine Polyanilin/Polyethylenoxid-Mischung; eine Polyvinylchlorid/Polyurethan-Mischung; eine Poly[(m-phenylenvinylen)-co-(2,5-dioctyloxy-p-phenylenvinylen)]/Polyethylenoxid-Mischung; eine Poly[2-methoxy-5-(2'-ethylhexyloxy)-l,4-phenylenvinylen] (MEH-PPV)/Polystyrol-Mischung; eine Polyanilin/Polystyrol-Mischung; eine Polyanilin/Polycarbonat-Mischung; eine Polyethylenterephthalat/Polyethylenterephthalat-co-polyethylenisophthalate-Mischung; eine Polysulfon/Polyurethan-Mischung; eine Chitosan/Polylactid-Mischung; eine Polyglykolid/Chitin-Mischung; eine Polylactid/Poly(lactid-co-glykolid)-Mischung; Polylactid-b-polyethylenoxid-Blockcopolymer; Poly(lactid-co-glykolid)-b-polyethylenoxid-Blockcopolymer; Poly[(trimethylencarbonat)-b-(B-caprolacton)]-Blockcopolymer; Polystyrol-b-polydimethylsiloxan-Blockcopolymer; Polystyrol-b-polypropylen-Blockcopolymer; Polystyrol-b-polybutadien-b-polystyrol-Blockcopolymer; Polystyrol-b-polyisopren-Blockcopolymer; eine Mischung aus Montmorillonit mit Polyamid-6, Polyamid-6,6, Polyvinylalkohol, Polymethylmethacrylat oder Polyurethan als Trägerstoff; eine Mischung aus einem Polymerträgerstoff und Edelmetallnanopartikeln; eine Mischung aus Polyacrylonitril-co-polyacrylsäure und Pd-Nanopartikeln; eine Mischung aus Polyethylenoxid und Au-Nanopartikeln; eine Mischung aus Polyvinylpyrrolidon und Ag-Nanopartikeln; eine Mischung aus Polyacrylonitril und Ag-Nanopartikeln; eine Mischung aus einem Polymerträgerstoff und magnetischen Nanopartikeln; eine Mischung aus Fe₃O₄-Nanopartikeln mit Polyethylenoxid oder Polyvinylalkohol; eine Mischung aus Poly(E-caprolacton) und FePt-Nanopartikeln; eine Mischung aus Polyurethan und MnZnNi-Nanopartikeln; eine Mischung aus Polymethylmethacrylat und Co-Nanopartikeln; eine Mischung aus einem Polymer und Kohlenstoffnanoröhrchen; Kohlenstoffnanoröhrchen gemischt mit Polyacrylonitril, Polyethylenoxid, Polyvinylalkohol, Polylactid, Polycarbonat, Polystyrol, Polyurethan oder Polymethylmethacrylat; eine Mischung aus einem Polymer und einem Metalloxid oder Metallsulfid; eine Mischung aus Polymer und TiO₂; eine Mischung aus einem TiO₂ und einem Polymer, ausgewählt aus Polyvinylpyrrolidon, Polyvinylacetat und Polyacrylonitril; eine Mischung aus Polymer und ZrO₂, eine Mischung aus ZrO₂ und einem Polymer, ausgewählt aus Polyvinylpyrrolidon, Polyvinylacetat und Polyvinylalkohol; oder eine Mischung aus a Polymer mit einem von ZnO, CuO, NiO, CeO₂, Mn₃O₄, Mn₂O₃/Mn₃O₄, MoO₃, BaTiO₃, Y₂O₃, Gd₂O₃, Ta₂O₅, Co₃O₄, Ba_{0,6}Sr_{0,4}TiO₃, SiO₂, CdS, PbS und Ag₂S umfassen.

5. Multiwell-Assayplatte nach einem der vorhergehenden Ansprüche, wobei die Polymernanofasern Poly(L-lactid) umfassen.

6. Multiwell-Assayplatte nach einem der vorhergehenden Ansprüche, wobei die Multiwell-Platte Folgendes ist:
eine 96-Well-Platte, wobei die Probenvertiefungen in einem Array von 12 x 8 Wells angeordnet sind;
eine 384-Well-Platte, wobei die Probenvertiefungen in einem Array von 24 x 16 Wells angeordnet sind;
eine 1536-Well-Platte, wobei die Probenvertiefungen in einem Array von 48 x 32 Wells angeordnet sind; oder
eine 3456-Well-Platte, wobei die Probenvertiefungen in einem Array von 72 x 48 Wells angeordnet sind.

7. Verfahren zur Herstellung einer Multiwell-Assayplatte, wobei die Multiwell-Assayplatte Folgendes umfasst:
eine transparente Plattenbasis, die den Boden mehrerer Probenvertiefungen definiert;
eine Gerüstschicht, die auf der Plattenbasis angeordnet ist, wobei die Gerüstschicht ein poröses, dreidimensionales Netzwerk an elektrogesponnenen Polymernanofasern in jeder der Probenvertiefungen bereitstellt, wobei die Dicke der Gerüstschicht in jeder der Probenvertiefungen von 10 µm bis 150 µm beträgt, und wobei der mittlere Durchmesser der Polymernanofasern von 500 nm bis 10 µm beträgt; und
einen Plattenrahmen, der die Seitenwände der Probenvertiefungen definiert;
wobei der Plattenrahmen undurchsichtig und durch die Gerüstschicht auf der Plattenbasis angeschweißt ist; wobei das Verfahren Folgendes umfasst:
(a) Anordnen einer Gerüstschicht zwischen einem undurchsichtigen Plattenrahmen und einer transparenten Plattenbasis, wobei der Plattenrahmen Seitenwände für mehrere Probenvertiefungen definiert, die Plattenbasis einen Boden für die Probenvertiefungen definiert, und die Gerüstschicht ein poröses, dreidimensionales Netzwerk von elektrogesponnenen Polymernanofasern umfasst, wobei die Dicke der Gerüstschicht von 10 µm bis 150 µm, und der mittlere Durchmesser der Polymernanofasern von 500 nm bis 10 µm beträgt; und
(b) Anschweißen des Plattenrahmens durch die Gerüstschicht an die Plattenbasis, mittels Laserschweißen oder Ultraschallschweißen, um eine wasserdichte oder hermetische Abdichtung zwischen dem Plattenrahmen und der Plattenbasis zu bilden.

8. Verfahren nach Anspruch 7, wobei das Schweißen (b) Folgendes umfasst:
Erwärmen des Plattenrahmens durch Aussetzen gegenüber einem Laser oder durch Ultraschallenergie, und somit Schmelzen des Plattenrahmens am Berührungspunkt zwischen dem Plattenrahmen und der Gerüstschicht; und
abkühlen lassen des geschmolzenen Materials, um eine Schweißnaht zwischen dem Plattenrahmen und der Plattenbasis zu bilden.

9. Verfahren nach Anspruch 8, wobei das Erwärmen sowohl den Plattenrahmen als auch die Gerüstschicht am Berührungspunkt zwischen dem Plattenrahmen und der Gerüstschicht schmilzt, und wobei das auskühlen lassen des geschmolzenen Materials eine Schweißnaht zwischen dem Plattenrahmen und der Plattenbasis bildet.

10. Verfahren nach einem der Ansprüche 7 bis 9, wobei das Verfahren Folgendes umfasst
(a1) in Kontakt bringen einer Gerüstschicht mit einem undurchsichtigen Plattenrahmen und einer transparenten Plattenbasis, wobei die Gerüstschicht zwischen dem Plattenrahmen und der Plattenbasis angeordnet ist, wobei der Plattenrahmen Seitenwände für mehrere Probenvertiefungen definiert, die Plattenbasis einen Boden für die Probenvertiefungen definiert, und die Gerüstschicht ein poröses, dreidimensionales Netzwerk von elektrogesponnenen Polymernanofasern umfasst, wobei die Dicke der Gerüstschicht von 10 µm bis 150 µm, und der mittlere Durchmesser der Polymernanofasern von 500 nm bis 10 µm beträgt;
(a2) Auftragen einer strukturierten Fotomaske an der Außenseite der Plattenbasis, und Anwenden einer Kraft, um die Gerüstschicht und Plattenbasis zwischen dem Plattenrahmen und der strukturierten Fotomaske zu halten; und
(b1) Aussetzen der strukturierten Fotomaske gegenüber einem Licht, umfassend das Aussetzen der strukturierten Fotomaske gegenüber einem Laser, und somit Erwärmen und Schmelzen des Plattenrahmens am Berührungspunkt zwischen dem Plattenrahmen und der Gerüstschicht,
wobei die strukturierte Fotomaske das Licht im Wesentlichen daran hindert, Bereiche der Gerüstschicht zwischen den Seitenwänden zu erreichen, und wobei die Plattenbasis dem Licht gegenüber transparent ist; und
(b2) abkühlen lassen des geschmolzenen Materials, um durch die Gerüstschicht eine Schweißnaht zwischen dem Plattenrahmen und der Plattenbasis zu bilden,
wobei das Verfahren wahlweise weiterhin Folgendes umfasst:
(b3) Entfernen der strukturierten Fotomaske.

11. Verfahren nach einem der Ansprüche 7 bis 9, wobei das Verfahren Folgendes umfasst:
(a1) in Kontakt bringen einer Gerüstschicht mit einem undurchsichtigen Plattenrahmen und einer transparenten Plattenbasis, wobei die Gerüstschicht zwischen dem Plattenrahmen und der Plattenbasis angeordnet ist, wobei der Plattenrahmen Seitenwände für mehrere Probenvertiefungen definiert, die Plattenbasis einen Boden für die Probenvertiefungen definiert, und die Gerüstschicht ein poröses, dreidimensionales Netzwerk von elektrogesponnenen Polymernanofasern umfasst, wobei die Dicke der Gerüstschicht von 10 µm bis 150 µm, und der mittlere Durchmesser der Polymernanofasern von 500 nm bis 10 µm beträgt;
(a2) Auftragen einer Schutzschicht an der Außenseite der Plattenbasis;
(a3) Auftragen einer strukturierten Fotomaske an der Außenseite der Schutzschicht, und Anwenden einer Kraft, um die Gerüstschicht, Plattenbasis und Schutzschicht zwischen dem Plattenrahmen und der strukturierten Fotomaske zu halten; und
(b1) Aussetzen der strukturierten Fotomaske gegenüber einem Licht, umfassend das Aussetzen der strukturierten Fotomaske gegenüber einem Laser, und somit Erwärmen und Schmelzen des Plattenrahmens am Berührungspunkt zwischen dem Plattenrahmen und der Gerüstschicht,
wobei die strukturierte Fotomaske das Licht im Wesentlichen daran hindert, Bereiche der Gerüstschicht, die zwischen den Seitenwänden angeordnet sind zu erreichen, und wobei die Plattenbasis und die Schutzschicht dem Licht gegenüber transparent sind; und
(b2) abkühlen lassen des geschmolzenen Materials, um durch die Gerüstschicht eine Schweißnaht zwischen dem Plattenrahmen und der Plattenbasis zu bilden,
wobei das Verfahren wahlweise weiterhin Folgendes umfasst:
(b3) Entfernen der strukturierten Fotomaske und der Schutzschicht.

12. Verfahren nach Anspruch 10 oder Anspruch 11, wobei der Laser ein Infrarotlaser oder ein Nah-Infrarotlaser ist.

13. Verfahren nach einem der Ansprüche 10 bis 12, wobei der Laser eine Wellenlänge von 810 nm bis 1064 nm aufweist.

14. Verfahren nach einem der Ansprüche 10 bis 13, wobei der Schritt des Aussetzens der strukturierten Fotomaske gegenüber einem Laser das Scannen eines Laserstrahls über die exponierte Oberfläche der Fotomaske umfasst.

15. Verfahren nach einem der Ansprüche 7 bis 14, weiterhin umfassend die Herstellung der Gerüstschicht, die in Schritt (a) verwendet wird, durch Elektrospinnen einer Nanofaservorgängerlösung auf ein Sammelsubstrat, wobei die Nanofaservorgängerlösung das Polymer in einem Lösemittel gelöst umfasst.

## Revendications

1. Plaque de dosage multipuits comprenant :
une base de plaque transparente qui définit le fond d'une pluralité de puits à échantillons ;
une couche formant échafaudage disposée sur la base de plaque, laquelle couche formant échafaudage fournit un réseau tridimensionnel poreux de nanofibres polymères électrofilées dans chacun desdits puits à échantillons, dans laquelle l'épaisseur de la couche formant échafaudage dans chacun desdits puits à échantillons est de 10 µm à 150 µm et le diamètre moyen des nanofibres polymères est de 500 nm à 10 µm ; et
un cadre de plaque, qui définit les parois latérales desdits puits à échantillons ;
dans laquelle le cadre de plaque est opaque et est soudé à la base de plaque au travers de la couche formant échafaudage.

2. Plaque de dosage multipuits selon la revendication 1 dans laquelle le diamètre moyen des nanofibres polymères est de 1 µm à 5 µm.

3. Plaque de dosage multipuits selon la revendication 1 ou la revendication 2 dans laquelle l'écart-type relatif par rapport à ladite moyenne est inférieur ou égal à 15 %.

4. Plaque de dosage multipuits selon l'une quelconque des revendications 1 à 3 dans laquelle les nanofibres polymères comprennent du poly(L-lactide) ; du poly(acide glycolique) ; du polyhydroxybutyrate ; du polystyrène ; du polyéthylène ; du polypropylène ; du poly(oxyde d'éthylène) ; un poly(uréthane ester) ; du poly(alcool de vinyle) ; du polyacrylonitrile ; du polylactide ; du polyglycolide ; du polyuréthane ; du polycarbonate ; du polyimide ; du polyamide ; du polyamide aliphatique ; du polyamide aromatique ; du polybenzimidazole ; du poly(téréphtalate d'éthylène) ; du poly[éthylène-co-(acétate de vinyle)] ; du poly(chlorure de vinyle) ; du poly(méthacrylate de méthyle) ; du poly(butyral de vinyle) ; du poly(fluorure de vinylidène) ; du poly(fluorure de vinylidène-co-hexafluoropropylène) ; de l'acétate de cellulose ; du poly(acétate de vinyle) ; du poly(acide acrylique) ; du poly(acide méthacrylique) ; du polyacrylamide ; de la polyvinylpyrrolidone ; du poly(sulfure de phénylène) ; de l'hydroxypropylcellulose ; du chlorure de polyvinylidène ; du polytétrafluoroéthylène ; un polyacrylate ; un polyméthacrylate ; un polyester ; une polysulfone ; une polyoléfine ; un polysilsesquioxane ; une silicone ; une résine époxy ; un ester de cyanate ; un polymère bis-maléimide ; une polycétone ; un polyéther ; une polyamine ; un polyphosphazène ; un polysulfure ; un polymère hybride organique/inorganique de ceux-ci, un copolymère de ceux-ci ou un mélange de ceux-ci ; un poly(lactide-co-glycolide) ; une polylactide-co-poly(ε-caprolactone) ; une poly(L-lactide)-co-poly(ε-caprolactone) ; un mélange de poly(alcool de vinyle) et de poly(acide acrylique) ; du collagène ; un mélange de collagène et de poly(oxyde d'éthylène) ; un mélange de collagène et de poly(ε-caprolactone) ; un mélange de collagène et de polylactide-co-poly(ε-caprolactone) ; de la gélatine ; un mélange de gélatine et de poly(ε-caprolactone) ; un mélange de gélatine et de poly(oxyde d'éthylène) ; un mélange de caséine et de poly(alcool de vinyle) ; un mélange de caséine et de poly(oxyde d'éthylène) ; une lipase ; un mélange de cellulose et de poly(alcool de vinyle) ; un mélange d'albumine de sérum bovin et de poly(alcool de vinyle) ; un mélange de luciférase et de poly(alcool de vinyle) ; de la α-chymotrypsine ; du fibrinogène ; de la soie ; de la soie régénérée ; de la soie de Bombyx mori régénérée ; un mélange de soie de Bombyx mori et de poly(oxyde d'éthylène) ; une fibroïne de soie ; un mélange de fibroïne de soie et de chitosan ; un mélange de fibroïne de soie et de chitine ; un mélange de soie et de poly(oxyde d'éthylène) ; de la soie d'araignée artificielle ; de la chitine ; du chitosan ; un mélange de chitosan et de poly(oxyde d'éthylène) ; un mélange de chitosan et de poly(alcool de vinyle) ; un mélange de chitosan quaternisé et de poly(alcool de vinyle) ; un mélange d'hexanoylchitosan et de polylactide ; de la cellulose ; de l'acétate de cellulose ; un mélange de polyvinylpyrrolidone/polylactide ; un mélange de polyaniline/polystyrène ; un mélange de polyaniline/poly(oxyde d'éthylène) ; un mélange de poly(chlorure de vinyle)/polyuréthane ; un mélange de poly[(m-phénylène vinylène)-co-(2,5-dioctyloxy-p-phénylène vinylène)]/poly(oxyde d'éthylène) ; un mélange de poly[2-méthoxy-5-(2'-éthylhexyloxy)-1,4-phénylène vinylène] (MEH-PPV)/polystyrène ; un mélange de polyaniline/polystyrène ; un mélange de polyaniline/polycarbonate ; un mélange de poly(téréphtalate d'éthylène)/poly(téréphtalate d'éthylène)-co-poly(isophtalate d'éthylène) ; un mélange de polysulfone/polyuréthane ; un mélange de chitosan/polylactide ; un mélange de polyglycolide/chitine ; un mélange de polylactide/poly(lactide-co-glycolide) ; un copolymère séquencé de polylactide-b-poly(oxyde d'éthylène) ; un copolymère séquencé de poly(lactide-co-glycolide)-b-poly(oxyde d'éthylène) ; un copolymère séquencé de poly[(carbonate de triméthylène)-b-(ε-caprolactone)] ; un copolymère séquencé de polystyrène-b-polydiméthylsiloxane ; un copolymère séquencé de polystyrène-b-polypropylène ; un copolymère séquencé de polystyrène-b-polybutadiène-b-polystyrène ; un copolymère séquencé de polystyrène-b-polyisoprène ; un mélange de montmorillonite avec un polyamide 6, un polyamide 6,6, du poly(alcool de vinyle), du poly(méthacrylate de méthyle) ou du polyuréthane comme matériau de support ; un mélange d'un support polymère et de nanoparticules de métal noble ; un mélange de poly(acrylonitrile)-co-poly(acide acrylique) et de nanoparticules de Pd ; un mélange de poly(oxyde d'éthylène) et de nanoparticules d'Au ; un mélange de polyvinylpyrrolidone et de nanoparticules d'Ag ; un mélange de poly(acrylonitrile) et de nanoparticules d'Ag ; un mélange d'un support polymère et de nanoparticules magnétiques ; un mélange de nanoparticules de Fe₃O₄ avec du poly(oxyde d'éthylène) ou du poly(alcool de vinyle) ; un mélange de poly(ε-caprolactone) et de nanoparticules de FePt ; un mélange de polyuréthane et de nanoparticules de MnZnNi ; un mélange de poly(méthacrylate de méthyle) et de nanoparticules de Co ; un mélange d'un polymère et de nanotubes de carbone ; des nanotubes de carbone mélangés avec du poly(acrylonitrile), du poly(oxyde d'éthylène), du poly(alcool de vinyle), du polylactide, du polycarbonate, du polystyrène, du polyuréthane ou du poly(méthacrylate de méthyle) ; un mélange d'un polymère et d'un oxyde de métal ou d'un sulfure de métal ; un mélange de polymère et de TiO₂ ; un mélange d'un TiO₂ et d'un polymère choisi parmi la polyvinylpyrrolidone, le poly(acétate de vinyle) et le poly(acrylonitrile) ; un mélange de polymère et de ZrO₂ ; un mélange de ZrO₂ et d'un polymère choisi parmi la polyvinylpyrrolidone, le poly(acétate de vinyle) et le poly(alcool de vinyle) ; ou un mélange d'un polymère avec l'un quelconque parmi ZnO, CuO, NiO, CeO₂, Mn₃O₄, Mn₂O₃/Mn₃O₄, MoO₃, BaTiO₃, Y₂O₃, Gd₂O₃, Ta₂O₅, Co₃O₄, Ba_{0.6}Sr_{0.4}TiO₃, SiO₂, CdS, PbS et Ag₂S.

5. Plaque de dosage multipuits selon l'une quelconque des revendications précédentes, dans laquelle les nanofibres polymères comprennent du poly(L-lactide).

6. Plaque de dosage multipuits selon l'une quelconque des revendications précédentes, dans laquelle la plaque multipuits est :
une plaque à 96 puits dans laquelle les puits à échantillons sont agencés en un réseau de 12 x 8 puits ;
une plaque à 384 puits dans laquelle les puits à échantillons sont agencés en un réseau de 24 x 16 puits ;
une plaque à 1536 puits dans laquelle les puits à échantillons sont agencés en un réseau de 48 x 32 puits ; ou
une plaque à 3456 puits dans laquelle les puits à échantillons sont agencés en un réseau de 72 x 48 puits.

7. Procédé de fabrication d'une plaque de dosage multipuits, laquelle plaque de dosage multipuits comprend :
une base de plaque transparente, qui définit le fond d'une pluralité de puits à échantillons ;
une couche formant échafaudage disposée sur la base de plaque, laquelle couche formant échafaudage fournit un réseau tridimensionnel poreux de nanofibres polymères électrofilées dans chacun desdits puits à échantillons,
dans lequel l'épaisseur de la couche formant échafaudage dans chacun desdits puits à échantillons est de 10 µm à 150 µm et le diamètre moyen des nanofibres polymères est de 500 nm à 10 µm ; et
un cadre de plaque, qui définit les parois latérales desdits puits à échantillons ;
dans lequel le cadre de plaque est opaque et est soudé à la base de plaque au travers de la couche formant échafaudage ;
lequel procédé comprend :
(a) la disposition d'une couche formant échafaudage entre un cadre de plaque opaque et une base de plaque transparente, dans lequel le cadre de plaque définit des parois latérales pour une pluralité de puits à échantillons, la base de plaque définit un fond pour ladite pluralité de puits à échantillons, et la couche formant échafaudage comprend un réseau tridimensionnel poreux de nanofibres polymères électrofilées, dans lequel l'épaisseur de la couche formant échafaudage est de 10 µm à 150 µm et le diamètre moyen des nanofibres polymères est de 500 nm à 10 µm ; et
(b) le soudage du cadre de plaque à la base de plaque, au travers de la couche formant échafaudage, par soudage au laser ou par soudage aux ultrasons, pour former un joint étanche à l'eau ou hermétique entre le cadre de plaque et la base de plaque.

8. Procédé selon la revendication 7, dans lequel ledit soudage (b) comprend :
le chauffage du cadre de plaque, par exposition de celui-ci à un laser ou par énergie ultrasonore, et ainsi la fusion du cadre de plaque au point de contact entre le cadre de plaque et la couche formant échafaudage ; et
le fait de laisser le bain de fusion refroidir pour former une soudure entre le cadre de plaque et la base de plaque.

9. Procédé selon la revendication 8, dans lequel ledit chauffage fait fondre à la fois le cadre de plaque et la couche formant échafaudage au point de contact entre le cadre de plaque et la couche formant échafaudage, et dans lequel le fait de laisser le bain de fusion refroidir permet de former une soudure entre le cadre de plaque et la base de plaque.

10. Procédé selon l'une quelconque des revendications 7 à 9, dans lequel le procédé comprend
(a1) la mise en contact d'une couche formant échafaudage avec un cadre de plaque opaque et une base de plaque transparente, dans lequel la couche formant échafaudage est disposée entre le cadre de plaque et la base de plaque, dans lequel le cadre de plaque définit des parois latérales pour une pluralité de puits à échantillons, la base de plaque définit un fond pour ladite pluralité de puits à échantillons, et la couche formant échafaudage comprend un réseau tridimensionnel poreux de nanofibres polymères électrofilées, dans lequel l'épaisseur de la couche formant échafaudage est de 10 µm à 150 µm et le diamètre moyen des nanofibres polymères est de 500 nm à 10 µm ;
(a2) l'application d'un masque photographique à motifs sur la partie extérieure de la base de plaque et l'application d'une force pour maintenir la couche formant échafaudage et la base de plaque entre le cadre de plaque et le masque photographique à motifs ; et
(b1) l'exposition du masque photographique à motifs à la lumière, comprenant l'exposition du masque photographique à un laser, et ainsi le chauffage et la fusion dudit cadre de plaque au point de contact entre le cadre de plaque et la couche formant échafaudage,
dans lequel le masque photographique à motifs empêche sensiblement ladite lumière d'atteindre des zones de la couche formant échafaudage entre les parois latérales, et dans lequel la base de plaque est transparente à ladite lumière ; et
(b2) le fait de laisser le bain de fusion refroidir pour former une soudure entre le cadre de plaque et la base de plaque au travers de la couche formant échafaudage,
éventuellement dans lequel le procédé comprend en outre :
(b3) le retrait du masque photographique à motifs.

11. Procédé selon l'une quelconque des revendications 7 à 9, dans lequel le procédé comprend :
(a1) la mise en contact d'une couche formant échafaudage avec un cadre de plaque opaque et une base de plaque transparente, dans lequel la couche formant échafaudage est disposée entre le cadre de plaque et la base de plaque, dans lequel le cadre de plaque définit des parois latérales pour une pluralité de puits à échantillons, la base de plaque définit un fond pour ladite pluralité de puits à échantillons, et la couche formant échafaudage comprend un réseau tridimensionnel poreux de nanofibres polymères électrofilées, dans lequel l'épaisseur de la couche formant échafaudage est de 10 µm à 150 µm et le diamètre moyen des nanofibres polymères est de 500 nm à 10 µm ;
(a2) l'application d'une couche protectrice sur la partie extérieure de la base de plaque ;
(a3) l'application d'un masque photographique à motifs sur la partie extérieure de la couche protectrice et l'application d'une force pour maintenir la couche formant échafaudage, la base de plaque et la couche protectrice entre le cadre de plaque et le masque photographique à motifs ; et
(b1) l'exposition du masque photographique à motifs à la lumière,
comprenant l'exposition du masque photographique à un laser, et ainsi le chauffage et la fusion dudit cadre de plaque au point de contact entre le cadre de plaque et la couche formant échafaudage,
dans lequel le masque photographique à motifs empêche sensiblement ladite lumière d'atteindre des zones de la couche formant échafaudage disposées entre les parois latérales, et dans lequel la base de plaque et la couche protectrice sont transparentes à ladite lumière ; et
(b2) le fait de laisser le bain de fusion refroidir pour former une soudure entre le cadre de plaque et la base de plaque au travers de la couche formant échafaudage,
éventuellement dans lequel le procédé comprend en outre :
(b3) le retrait du masque photographique à motifs et de la couche protectrice.

12. Procédé selon la revendication 10 ou la revendication 11, dans lequel le laser est un laser infrarouge ou un laser proche infrarouge.

13. Procédé selon l'une quelconque des revendications 10 à 12, dans lequel ledit laser a une longueur d'onde de 810 nm à 1064 nm.

14. Procédé selon l'une quelconque des revendications 10 à 13, dans lequel l'étape d'exposition du masque photographique à un laser comprend le balayage d'un faisceau laser sur toute la surface exposée du masque photographique.

15. Procédé selon l'une quelconque des revendications 7 à 14, qui comprend en outre la fabrication de la couche formant échafaudage utilisée à l'étape (a) par filage électrostatique d'une solution de précurseur de nanofibres sur un substrat de collecte, dans lequel la solution de précurseur de nanofibres comprend ledit polymère dissous dans un solvant.
